# EUROPEAN PATENT APPLICATION

(11) **EP 2 138 572 A1**
(43) Date of publication of application: **30.12.2009**
(21) Application number: 08011587.6
(22) Date of filing: 26.06.2008
(51) Int. Cl.: C12N 5/08, A61K 35/14, A61P 9/10

(54) **Mesoangioblast-like cell as well as methods and uses relating thereto**

(71) Applicant: t2cure GmbH, 60325 Frankfurt am Main (DE)
(72) Inventor: The designation of the inventor has not yet been filed
(74) Representative: Bösl, Raphael Konrad

(57) **Abstract**

The present invention relates to a medicament comprising a mesoangioblast-like cell obtained from a subject, a method of isolating a mesoangioblast-like cell, a method of producing a mesoderm-derived cell using a mesoangioblast-like cell, the use of a mesoangioblast-like cell for the preparation of a medicament for treating a cardiovascular disease and/or an ischemic disease and a method of converting the mesoangioblast-like cell into a pluripotent stem cell.

SEQUENCE LISTING

<110>  t2cure GmbH

<120>  Mesoangioblast-like cell as well as methods and uses relating
       thereto

<130>  T65599EP

<160>  84

<170>  PatentIn version 3.4

<210>  1
<211>  19
<212>  DNA
<213>  Artificial

<220>
<223>  Primer

<400>  1
gaaggtgaag gtcggagtc                                                    19


<210>  2
<211>  20
<212>  DNA
<213>  Artificial

<220>
<223>  Primer

<400>  2
gaagatggtg atgggatttc                                                   20


<210>  3
<211>  20
<212>  DNA
<213>  Artificial

<220>
<223>  Primer

<400>  3
cagaagactg tggatggccc                                                   20


<210>  4
<211>  20
<212>  DNA
<213>  Artificial

<220>
<223>  Primer

<400>  4
agtgtagccc aggatgccct                                                   20


<210>  5
<211>  20
<212>  DNA
<213>  Artificial

<220>
<223>  Primer

<400>  5
cagccctgat tcttccacca                                                    20


<210>  6
<211>  19
<212>  DNA
<213>  Artificial

<220>
<223>  Primer

<400>  6
gttcttgcat ctgctggag                                                     19


<210>  7
<211>  20
<212>  DNA
<213>  Artificial

<220>
<223>  Primer

<400>  7
gaacatgtgt aagctgcggc                                                    20


<210>  8
<211>  20
<212>  DNA
<213>  Artificial

<220>
<223>  Primer

<400>  8
ttctggcgcc ggttacagaa                                                    20


<210>  9
<211>  20
<212>  DNA
<213>  Artificial

<220>
<223>  Primer

<400>  9
cccacattaa tgaggcagcc                                                    20


<210>  10
<211>  20
<212>  DNA
<213>  Artificial

<220>
<223>  Primer

<400>  10
tgggggaagt cgcttcatgt                                                    20


<210>  11
<211>  20
<212>  DNA
<213>  Artificial

<220>
<223>  Primer

<400>  11
aacgacagca gctcgcccaa                                                   20


<210>  12
<211>  20
<212>  DNA
<213>  Artificial

<220>
<223>  Primer

<400>  12
ttgaggacca gtgggctgtg                                                   20


<210>  13
<211>  20
<212>  DNA
<213>  Artificial

<220>
<223>  Primer

<400>  13
atacgccagt gacgaccaga                                                   20


<210>  14
<211>  20
<212>  DNA
<213>  Artificial

<220>
<223>  Primer

<400>  14
tagttggggt ggtcctgcat                                                   20


<210>  15
<211>  23
<212>  DNA
<213>  Artificial

<220>
<223>  Primer

<400>  15
agatgcacaa ctcggagatc agc                                               23


<210>  16
<211>  24
<212>  DNA
<213>  Artificial

<220>
<223>  Primer

<400>  16
gccgttcatg tgcgcgtaac tgtc                                              24


<210>  17
<211>  24

<212>  DNA
<213>  Artificial

<220>
<223>  Primer

<400>  17
ccaggaaata cattgctgca caag                                              24


<210>  18
<211>  24
<212>  DNA
<213>  Artificial

<220>
<223>  Primer

<400>  18
tcgccttagc ttgacaacat aacc                                              24


<210>  19
<211>  20
<212>  DNA
<213>  Artificial

<220>
<223>  Primer

<400>  19
ctctgccaat ccatgtggga                                                   20


<210>  20
<211>  20
<212>  DNA
<213>  Artificial

<220>
<223>  Primer

<400>  20
cgtgcatgag acttcgatgc                                                   20


<210>  21
<211>  23
<212>  DNA
<213>  Artificial

<220>
<223>  Primer

<400>  21
tgatcgagcc actcctcaaa gag                                               23


<210>  22
<211>  23
<212>  DNA
<213>  Artificial

<220>
<223>  Primer

<400>  22
ctctttggaa ggtggattgc ctg                                               23

<210>  23
<211>  20
<212>  DNA
<213>  Artificial

<220>
<223>  Primer

<400>  23
atcagccagt gagggtcaac                                                    20


<210>  24
<211>  20
<212>  DNA
<213>  Artificial

<220>
<223>  Primer

<400>  24
ggccattctt ctcgtccata                                                    20


<210>  25
<211>  20
<212>  DNA
<213>  Artificial

<220>
<223>  Primer

<400>  25
accatcgagc tggagaagaa                                                    20


<210>  26
<211>  20
<212>  DNA
<213>  Artificial

<220>
<223>  Primer

<400>  26
tgctttgagg gttctcctgt                                                    20


<210>  27
<211>  24
<212>  DNA
<213>  Artificial

<220>
<223>  Primer

<400>  27
gaagaagaag aggaagcaaa ggag                                               24


<210>  28
<211>  19
<212>  DNA
<213>  Artificial

<220>
<223>  Primer

<400>  28
tccttctccc gctcattcc                                                    19


<210>  29
<211>  25
<212>  DNA
<213>  Artificial

<220>
<223>  Primer

<400>  29
agtgtaatca gtatttggac gagag                                             25


<210>  30
<211>  23
<212>  DNA
<213>  Artificial

<220>
<223>  Primer

<400>  30
tagcaggtcc gcaaggtgtg cag                                               23


<210>  31
<211>  20
<212>  DNA
<213>  Artificial

<220>
<223>  Primer

<400>  31
caccggccaa gtgtgcgtct                                                   20


<210>  32
<211>  18
<212>  DNA
<213>  Artificial

<220>
<223>  Primer

<400>  32
ccgcgttgtc cgcctctg                                                     18


<210>  33
<211>  19
<212>  DNA
<213>  Artificial

<220>
<223>  Primer

<400>  33
gacgggtcac tatctgtgc                                                    19


<210>  34
<211>  19
<212>  DNA
<213>  Artificial

<220>
<223>  Primer

<400>  34
cactacctga aggagctgc                                                    19


<210>  35
<211>  20
<212>  DNA
<213>  Artificial

<220>
<223>  Primer

<400>  35
ccacagctgg gagagggaat                                                   20


<210>  36
<211>  20
<212>  DNA
<213>  Artificial

<220>
<223>  Primer

<400>  36
caactccgtg cacagagtgg                                                   20


<210>  37
<211>  20
<212>  DNA
<213>  Artificial

<220>
<223>  Primer

<400>  37
catctggttg gttttcagcc                                                   20


<210>  38
<211>  21
<212>  DNA
<213>  Artificial

<220>
<223>  Primer

<400>  38
cggatgacaa agcaactatc c                                                 21


<210>  39
<211>  20
<212>  DNA
<213>  Artificial

<220>
<223>  Primer

<400>  39
agctgcaaca atggggagat                                                   20


<210>  40
<211>  20

<212>  DNA
<213>  Artificial

<220>
<223>  Primer

<400>  40
tgttgccaag cctcatagtg                                                    20


<210>  41
<211>  18
<212>  DNA
<213>  Artificial

<220>
<223>  Primer

<400>  41
gtcccggcag ctagagga                                                      18


<210>  42
<211>  20
<212>  DNA
<213>  Artificial

<220>
<223>  Primer

<400>  42
cctctgtctc ctcctcgtac                                                    20


<210>  43
<211>  20
<212>  DNA
<213>  Artificial

<220>
<223>  Primer

<400>  43
ccaccaggga ctcacaactt                                                    20


<210>  44
<211>  20
<212>  DNA
<213>  Artificial

<220>
<223>  Primer

<400>  44
gtagaggact tccgcagcac                                                    20


<210>  45
<211>  20
<212>  DNA
<213>  Artificial

<220>
<223>  Primer

<400>  45
atgcctttgg caaatacctg                                                    20

<210>  46
<211>  20
<212>  DNA
<213>  Artificial

<220>
<223>  Primer

<400>  46
ggtttcccat gttgcactct                                                    20


<210>  47
<211>  20
<212>  DNA
<213>  Artificial

<220>
<223>  Primer

<400>  47
ccaagctcag cacacaaaaa                                                    20


<210>  48
<211>  20
<212>  DNA
<213>  Artificial

<220>
<223>  Primer

<400>  48
ccaaccactc tgggaactgt                                                    20


<210>  49
<211>  20
<212>  DNA
<213>  Artificial

<220>
<223>  Primer

<400>  49
ttgtgatggg tgtgaaccac                                                    20


<210>  50
<211>  20
<212>  DNA
<213>  Artificial

<220>
<223>  Primer

<400>  50
ggatgcaggg atgatgttct                                                    20


<210>  51
<211>  20
<212>  DNA
<213>  Artificial

<220>
<223>  Primer

<400>  51
caccctgtgt ctcaacatgg                                                    20


<210>  52
<211>  20
<212>  DNA
<213>  Artificial

<220>
<223>  Primer

<400>  52
ggcttcaagg ttgtctctgg                                                    20


<210>  53
<211>  23
<212>  DNA
<213>  Artificial

<220>
<223>  Primer

<400>  53
acagccagta tgagatggac agc                                                23


<210>  54
<211>  23
<212>  DNA
<213>  Artificial

<220>
<223>  Primer

<400>  54
ccattggatg cctgcttctt cac                                                23


<210>  55
<211>  21
<212>  DNA
<213>  Artificial

<220>
<223>  Primer

<400>  55
gccgctggtg cacgtctctg c                                                  21


<210>  56
<211>  21
<212>  DNA
<213>  Artificial

<220>
<223>  Primer

<400>  56
ggcttccaga ggcagcggag g                                                  21


<210>  57
<211>  23
<212>  DNA
<213>  Artificial

<220>
<223>  Primer

<400>  57
gccccaacaa ggagaaagag tgc                                              23


<210>  58
<211>  23
<212>  DNA
<213>  Artificial

<220>
<223>  Primer

<400>  58
cgatgggtag tcgaattcgt tgc                                              23


<210>  59
<211>  23
<212>  DNA
<213>  Artificial

<220>
<223>  Primer

<400>  59
ccttacatta gcacaacacc agc                                              23


<210>  60
<211>  24
<212>  DNA
<213>  Artificial

<220>
<223>  Primer

<400>  60
tttttcagct ccttgtaaac aggc                                             24


<210>  61
<211>  24
<212>  DNA
<213>  Artificial

<220>
<223>  Primer

<400>  61
ggatcatttc tgggatccat atgc                                             24


<210>  62
<211>  23
<212>  DNA
<213>  Artificial

<220>
<223>  Primer

<400>  62
gcagatatac gtcccactgt ccg                                              23


<210>  63
<211>  24

<212>  DNA
<213>  Artificial

<220>
<223>  Primer

<400>  63
agaaagctct gagcatcgga tttg                                              24


<210>  64
<211>  23
<212>  DNA
<213>  Artificial

<220>
<223>  Primer

<400>  64
tgtaacctcc tgaagtgctg ctc                                               23


<210>  65
<211>  23
<212>  DNA
<213>  Artificial

<220>
<223>  Primer

<400>  65
catcgacgcc aaccacaaca tgc                                               23


<210>  66
<211>  23
<212>  DNA
<213>  Artificial

<220>
<223>  Primer

<400>  66
tgcacttcaa atgcgcaaca agc                                               23


<210>  67
<211>  23
<212>  DNA
<213>  Artificial

<220>
<223>  Primer

<400>  67
attccccaaa cctgaacacc agc                                               23


<210>  68
<211>  24
<212>  DNA
<213>  Artificial

<220>
<223>  Primer

<400>  68
caaaaatacc aatctattgt gggc                                              24

<210>  69
<211>  23
<212>  DNA
<213>  Artificial

<220>
<223>  Primer

<400>  69
atcaaaagga gtcggaaact ggc                                              23


<210>  70
<211>  23
<212>  DNA
<213>  Artificial

<220>
<223>  Primer

<400>  70
ttggtgcaag ctcttcaagg tgc                                              23


<210>  71
<211>  23
<212>  DNA
<213>  Artificial

<220>
<223>  Primer

<400>  71
aggtatgaga tcgtggtgga agc                                              23


<210>  72
<211>  24
<212>  DNA
<213>  Artificial

<220>
<223>  Primer

<400>  72
tggatgtatt cataatccag aggc                                             24


<210>  73
<211>  24
<212>  DNA
<213>  Artificial

<220>
<223>  Primer

<400>  73
aagatgccca gttttactgt gagc                                             24


<210>  74
<211>  23
<212>  DNA
<213>  Artificial

<220>
<223>  Primer

<400>  74
gttcctgtgg ttcactcagc agc                                              23


<210>  75
<211>  20
<212>  DNA
<213>  Artificial

<220>
<223>  Primer

<400>  75
tgctgcagct ctactctgga                                                  20


<210>  76
<211>  20
<212>  DNA
<213>  Artificial

<220>
<223>  Primer

<400>  76
ctgaggaggc gttcagaaac                                                  20


<210>  77
<211>  24
<212>  DNA
<213>  Artificial

<220>
<223>  Primer

<400>  77
gcctgaatga tgacattctt ttcg                                             24


<210>  78
<211>  23
<212>  DNA
<213>  Artificial

<220>
<223>  Primer

<400>  78
gcgaccctct gatgtcccaa gat                                              23


<210>  79
<211>  20
<212>  DNA
<213>  Artificial

<220>
<223>  Primer

<400>  79
gctgctgagt ggaagagagc                                                  20


<210>  80
<211>  20
<212>  DNA
<213>  Artificial

<220>
<223>  Primer

<400>  80
aattggccga gatccttctt                                                    20


<210>  81
<211>  20
<212>  DNA
<213>  Artificial

<220>
<223>  Primer

<400>  81
ttgccagcca ttatcattca                                                    20


<210>  82
<211>  20
<212>  DNA
<213>  Artificial

<220>
<223>  Primer

<400>  82
tatagagctg ctgcgggatt                                                    20


<210>  83
<211>  20
<212>  DNA
<213>  Artificial

<220>
<223>  Primer

<400>  83
gagaagggcg tgagagagtg                                                    20


<210>  84
<211>  20
<212>  DNA
<213>  Artificial

<220>
<223>  Primer

<400>  84
aaacagccag tgcaggagtt                                                    20

## Description

The present invention relates to a medicament comprising a mesoangioblast-like cell obtained from a subject's blood, a method of isolating a mesoangioblast-like cell, a method of producing a mesoderm-derived cell using a mesoangioblast-like cell, the use of a mesoangioblast-like cell for the preparation of a medicament for treating a cardiovascular disease and/or an ischemic disease and a method of converting the mesoangioblast-like cell into a pluripotent stem cell.

The use of stem cells has been hailed as the next major step in the battle against serious degenerative disorders, such as diabetes and cardiovascular diseases, and for some debilitating or lethal neurological diseases, such as Parkinson's and motor neuron disease. Medical researchers believe that stem cell therapy has the potential to dramatically change the treatment of human disease. The two broad types of mammalian stem cells are: embryonic stem cells that are found in blastocysts, and adult (or somatic) stem cells that are found in postnatal tissues. Although the number of human embryonic stem cell lines has increased considerably in the past years, few of these have been well characterized, and large hurdles still need to be overcome to ensure safety and efficacy. These will require substantial further investment and research. However, there exists a widespread controversy over human embryonic stem cell research that emanates from the techniques used in the creation and usage of stem cells. Human embryonic stem cell research is controversial because, with the present state of technology, starting a stem cell line requires the destruction of a human embryo and/or therapeutic cloning.

It is not the entire field of stem cell research, but the specific field of human embryonic stem cell research that is at the centre of an ethical debate. In contrast to embryonic stem cells, the use of adult (postnatal) or somatic stem cells is widely accepted from an ethnical point of view. A number of adult stem cell therapies already exist, particularly bone marrow transplants that are used to treat leukemia. In the future, medical researchers anticipate being able to use technologies derived from stem cell research to treat a wider variety of diseases including without limitation cancer, Parkinson's disease, spinal cord injuries, cardiovascular diseases and muscle damage, amongst a number of other impairments and conditions.

Also for cardiovascular regenerative medicine stem cell therapy offers novel treatment options. Several types of adult stem cells including different subsets of bone marrow-derived and tissue-resident progenitor cells were shown to induce vasculogenesis and cardiomyogenesis (Beltrami et al., Cell 114, 763-76 (2003), Orlic et al., Nature 410, 701-5 (2001)). However, the number, the proliferative capacity, and the angiogenic and cardiomyogenic potential of these progenitor cells are severely limited particularly in patients with cardiovascular diseases (Vasa et al., Circ Res 89, E1-7 (2001)).

Accordingly, there is a demand for an alternative source for adult stem cells. Preferably, these stem cells are to be isolated in a convenient, safe and economic manner. Additionally, it is preferred that these cells can be isolated from diseased patients, e.g. patients with cardiovascular diseases, particularly for allotransplantation.

Surprisingly, our studies identify a novel subset of circulating progenitor cells, which fulfill all criteria for an ideal cell population to be used, e.g. for cardiovascular regenerative therapeutic purposes: they are easily accessible in the peripheral blood, can be expanded *in vitro* and are capable of differentiating into distinct cell lineages *in vitro* and *in vivo* to large numbers. Additionally, hepatocyte growth factor (HGF) has been shown to mediate elevation of the level of these cells in the subject's blood. The identified cells resemble mesoangioblasts (MABs) and were therefore referred to as mesoangioblast-like cells (MAB-like cells). These MAB-like cells can be isolated from peripheral blood of children and adults.

Accordingly, a first aspect of the invention relates to a mesoangioblast-like cell, obtained from a subject's blood, as a medicament. In a particular embodiment of the present invention the subject is or has been exposed to hepatocyte growth factor (HGF) or an agent elevating the subject's HGF level, in order to increase the number of MAB-like cells in the subject's blood.

The marker profile of the novel cells is distinct from hematopoietic or mesenchymal stem cells, but resembles embryonic multipotent mesoangioblasts (MABs). Therefore, these cells have been designated with mesoangioblast-like cells (MAB-like cells). Mesoangioblasts (MAB) are vessel-associated cells identified during embryonic development. In contrast to hemangioblasts, MABs express mesenchymal (CD73) and endothelial markers, but lack the hematopoietic marker CD45. An MAB cell of the present invention is characterized by the presence of mesenchymal (e.g. CD73 and/or CD13) and endothelial (e.g. KDR, CD105 and/or VE-cadherin) markers, but lacks the hematopoietric factor CD45. Preferably, MAB-like cells are characterized by the absence of CD45 and the presence of CD73 and KDR. Additionally, the cells express one or more stem cell markers (e.g. islet-1) and are characterized by marked proliferative capacity and high telomerase activity at least at the beginning of their cultivation (e.g. until passage 30, 25, 20, 15, 10, especially until passage 15 or 10). Most preferably, MABs are characterized by the following marker profile:
- Surface markers: expression of KDR and CD73 and absence of CD45 (and optionally absence of CD34 and CD133); and
- Transcription factors: presence of Oct3/4, Klf4, c-myc and Sox 17.

By "high proliferative activity" is meant a population doubling within at most 50 hr, preferably at most 40 hr, most preferably at most 35 hrs. MAB-like cells are multipotent and improve functional recovery after ischemia.

Additionally, examined cells were shown to be capable of differentiating into distinct cell lineages, particularly distinct cardiovascular cell lineages, especially all 3 distinct cardiovascular cell lineages *in vitro* and *in vivo,* Furthermore, they may secrete pro-angiogenic and cardioprotective factors, and mediate functional improvements after therapeutic administration in models of ischemia and infarction. Phenotypically, these blood-derived cells may represent a correlate of embryonic dorsal aorta-derived mesoangioblasts present after birth and in the adult. Preliminary studies in children with previous gender-mismatched bone marrow-transplantation suggest that these circulating mesoangioblast-like cells are non-bone marrow-derived. Exposure of patients to cardiopulmonary bypass and cardioplegia during cardiac surgery is associated with mobilization of these mesoangioblast-like cells into the peripheral blood. The identification of HGF as a cytokine mediating mobilization of mesoangioblast-like cells into the peripheral blood not only offers the possibility to increase the number of circulating mesoangioblast-like cells, but opens up an avenue to generate patient-specific multipotent cells for therapeutic application in patients with cardiovascular disease.

Inventors identified circulating MAB-like cells in children. Children-derived MAB-like cells showed vigorous proliferation capacity and high telomerase activity. The capacity of children-derived MAB-like cells to acquire a cardiomyogenic phenotype has also been tested and confirmed. It is also shown that children-derived MAB-like cells express cardiac-specific genes after co-culture with cardiomyocytes and improved cardiac function *in vivo.* Since MAB-like cells can be easily isolated and expanded from peripheral blood, these cells are suitable to augment cardiac repair, e.g. in children with heart failure.

However, MAB-like cells may be also isolated from adults. The number and proliferative capacity of the cells is correlated with the donor age, but unexpectedly increased in patients undergoing extracorporal circulation. Hepatocyte growth factor (HGF), which is significantly elevated during extracorporal circulation, induces mobilization of mesoangioblast-like cells. Thus, HGF-mobilized clonally expandable, multipotent progenitor cells constitute a clinically useful source to generate subject- or patient-specific multipotent cells e.g. for therapeutic application in cardiovascular diseases.

Accordingly, the MAB-like cells may be used as a medicament, which may optionally encompass excipients and/or auxiliaries. It should be noted that a sufficient amount of MAB-like cells should be present in the medicament. It is also possible to combine the cells of two or more subjects into one medicament.

In a preferred embodiment the number of MAB-like cells to be administered to a subject amounts to at least 10 Mio, more preferable at least 20 Mio, still more preferably at least 50 Mio MAB-like cells per treatment. It might be necessary to administer the MAB-like cells in several doses, e.g. on different days for successful treatment.

In another preferred embodiment the cells are propagated before administration. For this, the MAB-like cells are preferably disaggregated into single clones which may be further expanded in e.g. liquid cultures containing suitable media, e.g. endothelial basal medium (EBM), X vivo 10 or X vivo 15 (e.g. from Biowhittaker), with supplements (e.g. hydrocortisone, bovine brain extract, antibiotics, growth factor(s) such as epidermal growth factor (EGF) or vascular endothelial growth factor (VEGF) and/or serum such as fetal calf serum, human serum or autologous serum). This is particularly useful, if larger amount of cells are needed for therapy or prophylaxis.

For administration the MAB-like cells should be in a pharmaceutical dosage form in general consisting of a mixture of ingredients known to a skilled person in the pharmacotechnical arts such as pharmaceutically acceptable excipients and/or auxiliaries combined to provide desirable characteristics. Examples of such substances are isotonic saline, Ringer's solution, buffers, medium (e.g. EBM, X vivo 10 and X vivo 15) organic or inorganic acids and bases as well as their salts and buffer solutions, sodium chloride, sodium hydrogencarbonate, sodium citrate or dicalcium phosphate, glycols, such a propylene glycol, sugars such as glucose, sucrose and lactose, starches such as corn starch and potato starch, albumins, organic solvents, complexing agents such as citrates and urea, stabilizers, such as protease or nuclease inhibitors, The physiological buffer solution preferably has a pH of approx. 6.0-8.0, especially a pH of approx. 6.8-7.8, in particular a pH of approx. 7.4, and/or an osmolarity of approx. 200-400 milliosmol/liter, preferably of approx. 290-310 milliosmol/liter. The pH of the pharmaceutical composition is in general adjusted using a suitable organic or inorganic buffer, such as, for example, preferably using a phosphate buffer, tris buffer (tris(hydroxyl-methyl)ami-nomethane). In general, the cells should be formulated and stored, e.g. by freezing, in order to facilitate viability of the cells by choosing appropriate conditions as known to the skilled person.

In addition to the capacity of the injected cells to contribute to tissue regeneration in its pure sense by virtue of their differentiation to the cardiovascular cell lineages, cells usually express and secrete a variety of pro-angiogenic cytokines and cardioprotective factors known to contribute to improved infarct healing. ELISA of insulin-like growth factor (IGF), stromal-derived factor-1 (SDF-1), vascular endothelial growth factor (VEGF), and hepatocyte growth factor (HGF) was significantly higher in cMAB supernatant.

Regeneration methods are well known in the art, and those skilled in the art are able to select an appropriate method for a desired purpose without undue experimentation. In an exemplified embodiment, the present cells are injected into a site of a recipient host, and let the cell stand for a certain period of time in order to regenerate into a tissue or organ. The medicament of the present invention can be administered to a subject by any route suitable for the administration of viable cells. Examples of such routes are intravascularly, intracranially, intracerebrally, intramuscularly, intradermally, intravenously, intraocularly, intraperitoneally, orthotopically in an injured organ or by open surgical procedure. The pharmaceutical composition may be administered to the subject by e.g. injection, infusion or implantation. It may be administered orthotopically, directly to the tissue or organ to be treated or reconstituted, i.e. the target tissue or target organ, or to a distant site. In one embodiment of the invention the medicament is injected into the peritoneum. Most preferably, the medicament (also referred to as pharmaceutical composition) is administered intravenously, intraperitoneally or orthotopically in an organ or tissue requiring repair or regeneration.

The term "transplantation" refers to a process where a cell, a tissue or an organ is removed from a donor or otherwise prepared from non-host source such as using genetic engineering techniques and implanted into a patient or recipient. The recipient may receive a cell, a tissue or an organ from a living-related donor (syngenic transplantation) or the recipient per se. The most compatible match is usually a sibling, as their genetic make-up may closely match. The transplantation may be syngeneic, allogeneic or xenogeneic. When syngeneic or xenogeneic transplantation is conducted, rejection response may optionally obviated by any method known in the art such as administering immunosuppressive agent (e. g. azathiopurine, cyclophosphamide etc.).

Accordingly, allotransplantation of cells is preferred. The present invention is particularly suitable for allotransplatation of MAB-like cells, wherein MAB-like cells are obtained from a subject, optionally propagated, genetically modified, converted into inducible pluripotent stem cells (iPSs; see below) and/or differentiated, and returned to the same subject in order to treat or prevent a disease or pathological condition, particularly a cardiovascular or ischemic disease.

The MAB-like cells may be used to prepare a medicament to be administered to a subject suffering a pathological condition or a disease, particularly a cardiovascular or ischemic disease. A pathological condition is any abnormal condition of the body of the subject.

In a preferred embodiment the pathological condition or disease is selected from the group consisting of from cancer, an autoimmune disease, a neurodegenerative disease, a respiratory disease, a vascular disease, diabetes mellitus, Alzheimer's disease, Lewy body dementia, Parkinson's disease, a trauma, burn, head trauma, spinal cord injury, stroke, myocardial infarction, arthrosis, Huntington's disease, Tourette's syndrome, multiple sclerosis, amyotrophic lateral sclerosis, Addison's disease, pituitary insufficiency, liver failure, inflammatory arthropathy, neuropathic pain, blindness, hearing loss, arthritis, a bacterial infection, a viral infection, a sexually transmitted disease and a damage of the skin, the eye, the nose, the ear, the brain, the spinal cord a nerve, the trachea, the lungs, the mouth, the esophagus, the stomach, the liver, the small intestines, the large intestines, the kidney, the ureter, the bladder, the urethra, a gland such as hypothalamus, pituitary, thyroid, pancreas and adrenal glands, the ovary, the oviduct, the uterus, the vagina, a mammary gland, the testes, the penis, a lymph nodes, a vessel, the heart, a blood vessel, a skeletal muscle, a smooth muscle, a bone, cartilage, a tendon or a ligament.

The MAB-like cell is isolated from a subject's blood. As used herein the term "subject" can mean either a human or non-human triploblastic animal, preferably mammals, especially primates, such as humans.

Hepatocyte growth factor/scatter factor (HGF/SF), which is a paracrine cellular growth, motility and morphogenic factor, may be used for mobilization of MAB-like cells. It is secreted by mesenchymal cells and targets and acts primarily upon epithelial cells and endothelial cells, but also acts on haemopoietic progenitor cells. It has been shown to have a major role in embryonic organ development, in adult organ regeneration and in wound healing. Hepatocyte growth factor regulates cell growth, cell motility, and morphogenesis by activating a tyrosine kinase signaling cascade after binding to the proto-oncogenic c-Met receptor. It is secreted as a single inactive polypeptide and is cleaved by serine proteases into a 69-kDa alpha-chain and 34-kDa beta-chain. A disulfide bond between the alpha and beta chains produces the active, heterodimeric molecule. The protein belongs to the plasminogen subfamily of S1 peptidases but has no detectable protease activity. Alternative splicing of this gene produces multiple transcript variants encoding different isoforms. However, the inactive pro-peptide as well as the splice variants may be used in the context of the present invention.

Alternatively, a HGF-elevating agent may be used in order to mediate mobilization of MAB-like cells into the subject's blood.

One example of an agent elevating the subject's HGF blood level is heparin or a functionally active derivative thereof, such as a low-molecular-weight heparin (LMWH; see below).

Heparin is a highly-sulfated glycosaminoglycan, which is widely used as an injectable anticoagulant and has a very highest negative charge density. Heparin is a member of the glycosaminoglycan family of carbohydrates (which includes the closely-related molecule heparan sulfate) and consists of a variably-sulfated repeating disaccharide unit. The most common disaccharide unit is composed of a 2-O-sulfated iduronic acid and 6-O-sulfated, N-sulfated glucosamine, IdoA(2S)-GlcNS(6S). Under physiological conditions, the ester and amide sulfate groups are deprotonated and attract positively-charged counterions to form a heparin salt. Natural heparin consists of molecular chains of varying lengths, or molecular weights. Chains of molecular weight usually range from 3000 to over 40,000 Daltons, making up polydisperse pharmaceutical-grade heparin. Pharmaceutical grade heparin is usually derived from mucosal tissues of slaughtered meat animals such as porcine intestine or bovine lung. However, the effects of natural or unfractionated heparin can be difficult to predict. After a standard dose of unfractionated heparin, coagulation parameters should be monitored very closely to prevent over- or under-anticoagulation. Therefore, the average molecular weight of most commercial heparin preparations is in the range of 12 kDa to 15 kDa.

One alternative is a functionally active derivative, especially fragment, of heparin. The derivate may be a chemically modified heparin and/or a heparin fragment. The functionally active derivative, especially fragment, of heparin is characterized by having a biological activity similar to that displayed by heparin, particularly the ability to induce mobilization of MAB-like cells. Particularly, the derivative of heparin is functionally active in the context of the present invention, if the activity of the derivative amounts to at least 10%, preferably at least 25%, more preferably at least 50%, even more preferably at least 70%, still more preferably at least 80%, especially at least 90%, particularly at least 95%, most preferably at least 99% of the activity of heparin.

The above disadvantage of natural or unfractionated heparin led to the development of heparin derivates, particularly low-molecular-weight heparins (LMWHs), which, in contrast, consist of only short chains of polysaccharide. LMWHs are defined as heparin salts having an average molecular weight of less than about 8000 Da and for which at least 60% of all chains have a molecular weight less than about 8000 Da. These are obtained by various methods of fractionation or depolymerisation of polymeric heparin. Various methods of heparin depolymerisation are used in the manufacture of low-molecular-weight heparin. Examples and resulting products, which can be used in the context of the present invention, are listed below:
- Oxidative depolymerisation with hydrogen peroxide. Used in the manufacture of ardeparin (Normiflo)
- Deaminative cleavage with isoamyl nitrite. Used in the manufacture of certoparin (Sandoparin)
- Alkaline beta-eliminative cleavage of the benzyl ester of heparin. Used in the manufacture of enoxaparin (Lovenox and Clexane)
- Oxidative depolymerisation with Cu²⁺ and hydrogen peroxide. Used in the manufacture of parnaparin (Fluxum)
- Beta-eliminative cleavage by the heparinase enzyme. Used in the manufacture of tinzaparin (Innohep and Logiparin)
- Deaminative cleavage with nitrous acid. Used in the manufacture of dalteparin (Fragmin), reviparin (Clivarin) and nadroparin (Fraxiparin)

A further functionally active heparin derivative is fondaparinux (Arixtra), which is a synthetic pentasaccharide, whose chemical structure is almost identical to the AT binding pentasaccharide sequence. Apart from the O-methyl group at the reducing end of the molecule, the identity and sequence of the five monomeric sugar units contained in fondaparinux is identical to a sequence of five monomeric sugar units that can be isolated after either chemical or enzymatic cleavage of the polymeric glycosaminoglycans heparin and heparan sulfate (HS). that can be found within polymeric heparin and heparan sulfate. One potential advantage of fondaparinux over LMWH or unfractionated heparin is that the risk for heparin-induced thrombocytopenia is substantially lower.

Further examples of agents elevating the subject's HGF level include Hepatocyte growth factor activator precursor (EC 3.4.21.-) (HGF activator; HGFA) including Hepatocyte growth factor activator short chain and Hepatocyte growth factor activator long chain.

The exposure to HGF or an HGF elevating agent may be done by applying the substance in question to the subject as known to the skilled practitioner. Methods of administration are detailed in the present description.

In a preferred embodiment of the invention, the mesoangioblast-like cell has been or is obtained from an adult's blood. An adult in the context is of present invention relates to a subject sexual and/or physical mature. Sexual maturity is the age/stage when an organism can reproduce. Physical maturity is the age/stage at which the subject reaches its maximum height and secondary sex characteristics (e.g. for humans form such as body hair and facial hair, voice lowers in pitch, and menses begin (women)). Especially, for isolation from adult's blood, the prior treatment with HGF or an HGF-elevating agent, particularly HGF, heparin or HGF activator, is envisioned, in order to increase the number of MAB-like cells in the adult's blood.

In another preferred embodiment of the invention, the mesoangioblast-like cell has been or is obtained from a mammal. Examples of mammals include without limitation rat, mouse, cat, dog, horse, pig, cow, rabbit, sheep, goat and particularly primates, especially humans. The human subject may be a child (postnatal being before adolescence as defined above) or an adult. In a particular preferred embodiment of the invention the child or adult is suffering from a cardiovascular disease requiring regenerative treatment, such as cardiac repair.

In a preferred embodiment of the invention the mesoangioblast-like cell may be characterized by the presence and/or absence of particular markers. The presence and absence of these markers may be determined using methods known to the skilled person such as FACS analysis, RT-PCR, immunostaining and/or cytochemical staining. The methods may be carried out e.g. as described in the Examples.

The feature negative for a marker refers to the identification of marker on the surface of cells using e.g. FACS analysis or RT-PCR as detailed in the Examples. Preferably, a MAB-like cell is marker negative, if the signal obtained with the respective detection method is below the threshold or not significantly different from the background or the negative control.

The feature positive for a marker refers to the identification of marker on the surface of cells using e.g. FACS analysis or RT-PCR as detailed in the Examples. Preferably, a MAB-like cell is marker positive, if the signal obtained with the respective detection method is above the threshold or significantly increased relative to the background or negative control.

As detailed above, the MAB cell is characterized by the presence of mesenchymal (e.g. CD73 and/or CD13) and endothelial (e.g. KDR, CD105 and/or VE-cadherin markers), but lacks the hematopoietric factor CD45.

The mesenchymal marker may be CD73 and/or CD13. Additionally, the following mesenchymal marker may be present: CD44. Particularly, the presence of the following combinations of these markers may be identified:
- CD73 and CD 13,
- CD73 and CD44,
- CD13 and CD44, or
- CD73, CD13 and CD44.

The endothelial marker may be KDR (CD309), CD105 and/or VE-cadherin (CD144). Particularly, the presence of the following combinations of these markers may be identified:
- KDR and CD105,
- CD105 and VE-cadherin,
- KDR and VE-cadherin, or
- KDR, CD105 and VE-cadherin.

As detailed above, the MAB-like cell is characterized the absence of CD45. Moreover, one or both of the following factors may be absent: CD34 and/or CD133. Particularly, the absence of the following combinations of these markers may be identified:
- CD45 and CD133,
- CD45 and CD34,
- CD45, CD34 and CD133.
   In a still more preferred embodiment of the present invention, the MAB-like cell is characterized by
- the presence of CD73 or KDR (VEGF-receptor-2), optionally also the presence of one or more markers selected from the group consisting of Oct3/4, Klf4, c-myc and Sox17; and the absence of CD45, optionally also the absence CD34 or CD133, or
- the presence of KDR, CD73 and CD29 (and optionally also the presence of Oct3/4, Klf4, c-myc and/or Sox17); and the absence of CD45, optionally also the absence CD34 or CD133, or
- the presence of CD73, CD13, CD44, CD144, KDR and CD105 and the absence of CD45, CD34 and CD133, or
- the presence of CD73, KDR and telomerase activity and the absence of CD45; or
- especially the presence of CD73 and KDR (VEGF-receptor-2), optionally also the presence CD44, Klf4, Oct3/4 and CD29, and the absence of CD45, optionally also the absence CD34 and CD133, or
- especially the presence of CD105, CD 144, KDR, CD 13, CD73, CD29 and CD44 and the absence of CD34, CD45, CD 133 and CD 18, or
- most preferably the presence of CD73, KDR (VEGF-receptor-2), Oct3/4, Klf4, c-myc and Sox17; and the absence of CD45, CD34 and CD133.

Additionally, the MAB-like cell may be characterized by the presence of one or more stem cell markers. An example of these markers includes islet-1. Furthermore, stem cell properties of the cells may be proven by high telomerase activity.

Suitable methods for determining the presence and absence of these factors and marker include flow cytometry, immunohistochemistry and RT-PCR and are described into more detail in the Examples.

In a still more preferred embodiment of the invention, the mesoangioblast-like cell is defined by one or more of the following:
i) the mesoangioblast-like cell has been isolated at least 1 h to 48 h after the subject's exposure to HGF and/or wherein the subject is exposed to 0.01 to 100 µg/kg bodyweight HGF;
ii) the mesoangioblast-like cell has been isolated at least 1 h to 48 h after the subject's exposure to heparin or a functionally active derivative thereof, e.g. a low-molecular-weight heparin, and/or wherein the subject is exposed to 0.1 to 10 mg/kg bodyweight heparin or a functionally active derivative thereof, e.g. a low-molecular-weight heparin; or
iii) the mesoangioblast-like cell has been isolated at least 1 h to 48 h after the subject's exposure to a combination of (i) HGF and (ii) heparin or a functionally active derivative thereof, e.g. a low-molecular-weight heparin, and/or wherein the subject is exposed to (i) 0.01 to 100 µg/kg bodyweight HGF and (ii) 0.1 to 10 mg/kg bodyweight heparin or a functionally active derivative thereof, e.g. a low-molecular-weight heparin.
   As detailed above, HGF or an HGF-elevating agent (such as heparin or a functionally active heparin derivative) may be used in order to mediate mobilization on MAB-like cells into the subject's blood. In order to obtain proper mobilization of MAB-like cells, the above regimen with respect to time and or concentration of the agent mobilizing MAB-like cells may be followed.

Particularly, for each of the above regimens i) to iii), the time between agent application and isolating of MAB-like cells may be further specified as 1 h to 72 h after the subject's exposure to the agent, particularly at least 2h to 48 h, preferably 2h to 36 h, more preferably 2h to 12 , even more preferentially 2-6 hours.

Alternatively or additionally, for each of the above regimens i) to iii), the concentration of the agent mobilizing MAB-like cells may be further specified as
- from about 0.01 to about 100 µg/kg bodyweight, particularly from about 0.1 to about 50 µg/kg bodyweight, more preferably from about 1 to about 30 µg/kg bodyweight (particularly for HGF), and/or
- from about 0.01 to about 100 µg/kg bodyweight, particularly from about 0.05 to about 30 µg/kg bodyweight, more preferably from about 0.1 to about 10 µg/kg bodyweight (particularly for heparin of the functionally active derivative thereof).

However, the skilled practitioner will understand that the above concentrations depend from a variety of factors (species, disease state, age, sex, body weight and difference in sensitivity of the administration object, timing and interval of administration, characteristics, dispensing and kind of the pharmaceutical preparation, the kind of the active ingredient and the like) and will have to be adapted to the prevailing circumstances.

In another preferred embodiment of the invention, the mesoangioblast-like cell is capable of differentiating into a mesoderm-derived cell, particularly into one, two or three cardiovascular lineage(s).

The mesoderm is a one of the three germ layers. During embryogenesis the zygote undergoes rapid cell divisions with no significant growth, producing a blastula. During gastrulation cells migrate to the interior of the blastula, consequently forming three (triploblastic) germ layers. The embryo during this process is called a gastrula. The germ layers are referred to as the ectoderm, mesoderm and endoderm. The germ layers eventually give rise to all of an animal's tissues and organs through the process of organogenesis. The mesoderm generally gives rise to the following organs or tissues bones, most of the circulatory system, including the heart and major blood vessels, connective tissues of the gut and integuments, mesenchyme, mesothelium, skeletal muscles, peritoneum (lining of the coelom), reproductive system, urinary system (including the kidneys). Accordingly, in a preferred embodiment of the present invention, the mesoangioblast-like cell is capable of differentiating into cells of at least one of these organs or tissues. More preferably, the mesoangioblast-like cell is capable of differentiating into one, two or three cardiovascular lineage(s). A cardiovascular lineage describes cardiovascular cells with a common ancestry, which is developing from the same type of identifiable immature progenitor cell. For example, the functional heart is comprised of distinct mesoderm-derived lineages including cardiomyocytes, endothelial cells and vascular smooth muscle cells. Studies in the mouse embryo and the mouse embryonic stem cell differentiation model have provided evidence indicating that these three cardiovascular lineages develop from a common cardiovascular progenitor that represents one of the earliest stages in mesoderm specification to the cardiovascular lineages. The capability of MAB-like cells to differentiate into several cardiovascular lineages identify MAB-like cells as a progenitor that defines one of the earliest stages of human cardiac development. Consistently, MAB-like cells express the marker KDR, which defines early cardiovascular progenitor cells in embryonic stem cells (Yang et al, Nature. 2008 May 22;453(7194):524-8).

In one embodiment of the invention the mesoangioblast-like cell may be differentiated into a mesoderm-derived cell, particularly a cardiovascular cell. These cells may be transplanted into the organ or tissue requiring repair or regeneration, e.g. the myocardium. "Differentiation" in the present context refers to a status of cells in which the cells develop specific morphological or functional properties. Cells may "differentiate" into a specific tissue or organ. In the context of cardiovascular cells, "differentiation" refers to develop at least one property of a cell of the cardiovascular system. Typical characteristics of these cells (e.g. smooth muscle cells, endothelial cells and cardiomyocytes) are detailed herein.

The term "organ" refers to two or more adjacent layers of tissue, which layers of tissue maintain some form of cell-cell and/or cell-matrix interaction to form a microarchitecture.

The term "tissue" refers to a group or layer of similarly specialized cells which together perform certain special functions.

In accordance with the present invention the mesoangioblast-like cell may be used for the treatment of a cardiovascular disease and/or an ischemic disease, particularly wherein the cardiovascular and/or ischemic disease is selected from the group consisting of myocardial infarction, heart failure and peripheral vascular occlusive disease. Cardiovascular diseases as well as ischemic diseases often lead to irreversible damages of organs and tissues involved.

The term "cardiovascular disease" refers to the class of diseases that involve the heart or blood vessels (arteries and veins). While the term technically refers to any disease that affects the cardiovascular system, it is usually used to refer to those related to atherosclerosis (arterial disease). These conditions have similar causes, mechanisms, and treatments.

An ischaemic or ischemic disease is a disease characterized by reduced oxygen supply to the an organ or tissue, usually due to coronary artery disease (atherosclerosis of the coronary arteries). Its risk increases with age, smoking, hypercholesterolemia (high cholesterol levels), diabetes, hypertension (high blood pressure).

Preferred examples include myocardial infarction, heart failure and peripheral vascular occlusive disease.

Another aspect of the present invention relates to a method of isolating a mesoangioblast-like cell from a subject, comprising the steps of:
a) optionally exposing a subject to HGF and/or an agent elevating the subject's HGF level; and
b) isolating a mesoangioblast-like cell mobilized by step a) from the subject's blood.

The features "MAB-like cell" and "subject" may be as defined above. Additionally, step a) of this method may be carried out as detailed above in the context of the MAB-like cell of the present invention.

After the subject has been exposed to the HGF and/or an agent elevating the subject's HGF level the MAB-like cell is isolated from the subject's blood. For this, a blood sample may be taken from the subject. Particularly for mammals, this may be conveniently performed by taking venous blood from the subject. Venous blood may be obtained by venipuncture from a the mammal, e.g. a human donor, wherein usually only a small sample, e.g. 50 ml to 100 ml sample, of blood is adequate for the method of the present invention (see Examples). Blood is most commonly obtained from the median cubital vein, on the anterior forearm (the side within the fold of the elbow). This vein lies close to the surface of the skin, and there is not a large nerve supply. Most blood collection in the industrialized countries is done with an evacuated tube system consisting of a plastic hub, a hypodermic needle, and a vacuum tube. However, blood may also be obtained by any other method known to the skilled person.

After isolation of the blood, MAB-like cells are isolated from the blood sample. These cells may be isolated on the basis of their characteristic marker profile, as defined above in the context of any of the embodiments of the present invention. This may be done with a fluorescence-activated cell sorting (FACS).

Alternatively, the isolation of MAB-like cells may be done as detailed in the Examples. For this, mononuclear cells (MNCs) may be isolated from blood by any suitable method known by the skilled person e.g. by density centrifugation as density gradient centrifugation using a Ficoll gradient. Thereafter, MNCs may be plated on suitable culture dishes, preferably coated with e.g. fibronectin, and maintained under suitable conditions, which induce the presence of supplemental growth factors. After a suitable time, e.g. after several days such as 7 days, non-adherent cells are discarded. Remaining cells are MAB-like cells (and EPC after 1^{st} cultivation). These cells may be used or further cultivated under suitable conditions for propagation or in order to obtain differentiated cells. Additionally or alternatively, cells might be sorted by using the marker combinations described above.

After having been obtained and optionally further purified, the cells may be immediately cultivated or frozen for storage as known to the person skilled in the art. The cells may be frozen at liquid nitrogen temperatures and stored for long periods of time, being thawed and capable of being reused. The cells will usually be stored in 10% DMSO, 70% autologous plasma (irradiated with 2500 rad), 20% culture medium. Cells may be frozen in a programmable cell freezer to -180° C in liquid nitrogen. Once thawed, the cells may be expanded by use of growth factors or cells associated with stem cell proliferation and differentiation.

The methods of the present invention may encompass cultivation of cells under suitable conditions. Suitable conditions include an appropriate temperature and gas mixture (typically, 37°C, 5% CO₂) in a cell incubator. Alternatively hypoxic incubators can be used. Aside from temperature and gas mixture, the most commonly varied factor in culture systems is the culture media including growth medium. The term "culture medium" is recognized in the art, and refers generally to any substance or preparation used for the cultivation of living cells. Recipes for growth media can vary in pH, glucose concentration, growth factors, and the presence of other nutrient components. The factors used to supplement media are often derived from animal blood, such as calf serum or by a feeder layer. Culture conditions may vary from species to species; however, typical conditions are the following are:
Exemplary procedures for isolating the cells of the invention are as follows: Blood samples may be collected, e.g. from the patients who undergo open heart surgery with cardiopulmonary bypass. Mononuclear cells (MNCs) may be isolated by Ficoll density gradient centrifugation with Biocoll separating solution (Biochrom AG, Berlin, Germany). MNCs are plated in endothelial basal medium (EBM) (CellSystems, St. Katharinen, Germany), with supplements (1 µg/ml hydrocortisone, 3 µg/ml bovine brain extract, 30 µg/ml gentamicin, 50 µg/ml amphotericin B, 10 µg/ml EGF, and 20% fetal calf serum) on culture dishes coated with human fibronectin (Sigma, St Louis, MO). After 7 days in culture, non-adherent cells are discarded and cells are cultured for additional 7 days in same medium. On day 15, cells are detached by 0.25% Trypsin-EDTA (GIBCO) and are seeded at 5×10⁴ cells/ml on fibronectin-coated dishs. When cells reached 80% confluency, cells are subsequently passaged at 5×10⁴ cells/ml and are used for following analyses.

Alternatively, X-vivo medium + autologous serum can be used. Mononuclear cells may be suspended in X vivo-15 medium (Biowhittaker) supplemented with 1 ng/mL carrier-free human recombinant VEGF (R&D), 20% human serum and potentially with 0.1 µmol/L atorvastatin from each individual patient. Cells are seeded at a density of 6.4x10⁵ cells/mm² at fibronectin-coated dishes (Roche).

Suitable conditions are also given in the Examples.

Still another aspect of the present invention relates to a method of producing a mesoderm-derived cell, comprising the steps of:
a) optionally exposing a subject to HGF and/or an agent elevating the subject's HGF level;
b) isolating a mesoangioblast-like cell mobilized by step a) from the subject; and
c) differentiating the mesoangioblast-like cell into a mesoderm-derived cell.

Steps a) and b) of the method of the invention of producing a mesoderm-derived cell may be carried out as detailed above in connection with the method of isolating a mesoangioblast-like cell from a subject.

In step c) the MAB-like cells are differentiated into a mesoderm-derived cell, e.g. by addition of suitable factors inducing differentiation into the desired mesoderm-derived cell. Examples of suitable mesoderm-derived cells include without limitation endothelial cell, a smooth muscle cell, a cardiomyocyte, an osteoblast, an pericyte, a fibroblast, and a myofibroblast.

In preferred embodiment of the invention the mesoangioblast-like cell may be further characterized as detailed above in the context of the MAB cells of the present invention. This is particularly true for the use of heparin as HGF elevating agent, for the characterization of the subject from which the MSB-like cell is derived, for the presence and/or absence of particular markers as defined above and for the conditions for exposure to an HGF elevating agent.

In a further embodiment of the invention the mesoderm-derived cell is an endothelial cell, a smooth muscle cell, a cardiomyocyte or an osteoblast.

In a further embodiment of the present invention, the above method comprises expanding, particularly clonally expanding the mesoangioblast-like cell isolated in step b).

In a preferred embodiment the differentiating of step c) may be carried out by incubating the mesoangioblast-like cell in the presence of a differentiation factor or a cell producing such factors (e.g. a cardiomyocyte for differentiation into cardiac cells (see Examples)). Suitable conditions for inducing differentiation into
- endothelial cells include the use of Matrigel Basement Membrane Matrix (BD Bioscience).
- smooth muscle cells include the presence of TGFβ or FGF8.
- cardiomyocytes include presence of neonatal cardiomyocytes.
- cardiomyocytes induced by Wnt3a and dexamethason.
- cardiomyocytes induced by Jagged-1.
- cardiomyocyte induced by Azacytidin.

Suitable conditions are also detailed in the Examples.

A further aspect of the present invention relates to the use of a mesoangioblast-like cell of the present invention for the preparation of a medicament for treating a cardiovascular disease and/or an ischemic disease, wherein the mesoangioblast-like cell has been obtained from a subject's blood, optionally after having been exposed to hepatocyte growth factor (HGF) and/or an agent elevating the subject's HGF level. If the medicament comprising the MAB-like cells is administered to the recipient, the cells will be applied to or migrate mainly to a target tissue or organ, e.g. an tissue or organ to be repaired. In the environment of this tissue or organ, new cells forming or regenerating the organ or tissue will be generated by differentiating the cells of the MAB-like cells into the respective cells, e.g. cardiomyocytes.

Still another aspect of the invention relates to a method of for treating a cardiovascular disease and/or an ischemic disease comprising administering to a subject an effective amount of MAB-like cells of the present invention. The specific therapeutically effective amount of cells for any particular subject will depend upon a variety of factors including the condition or disease the subject is suffering from, the route of administration, the age, body weight and sex of the patient, the duration of the treatment and like factors well known in the medical arts.

In preferred embodiment of the invention the mesoangioblast-like cell may be further characterized as detailed above in the context of the MAB cells of the present invention. This is particularly true for the use of heparine as HGF elevating agent, for the characterization of the subject from which the MSB-like cell is derived, for the presence and/or absence of particular markers as defined above, for the conditions for exposure to an HGF elevating agent, for the differentiation capacity, for the differentiation status and for its use as medicament, especially for the diseases as defined above.

A final aspect of the present invention relates to a method of converting the mesoangioblast-like cell of the present invention (as defined in any of the above embodiments) into an inducible pluripotent stem cell, wherein the level of Sox2 protein in the mesoangioblast-like cell is increased, particularly wherein the increase is mediated by:
a) transfecting the mesoangioblast-like cell of the present invention with the Sox2 gene and expressing the same;
b) transducing the mesoangioblast-like cell of the present invention with the Sox2 protein; and/or
c) activating a promoter directing the Sox2 gene expression in the mesoangioblast-like cell of the present invention.

Adult (or somatic) stem cells are cells found in most, if not all, multi-cellular organisms. They are characterized by the ability to renew themselves through mitotic cell division and differentiating into a diverse range of specialized cell types. In postnatal or adult organisms, stem cells and progenitor cells act as a repair system for the body, replenishing specialized cells, but also maintain the normal turnover of regenerative organs, such as blood, skin or intestinal tissues. Inducible pluripotent stem cells, commonly abbreviated as iPS cells or iPSCs, are a type of pluripotent stem cell artificially derived from a non-pluripotent cell, typically an adult somatic cell, by inserting certain genes. Induced pluripotent stem cells are believed to be identical to natural pluripotent stem cells, such as embryonic stem cells, in many respects such as the expression of certain stem cell genes and proteins, chromatin methylation patterns, doubling time, embryoid body formation, teratoma formation, viable chimera formation, and potency and differentiability, but the full extent of their relation to natural pluripotent stem cells is still being assessed. Inducable pluripotent stem cells are in general generated by the following method: (1) Isolation and cultivation of donor cells. (2) Transfection of cells with stem cell-associated genes (e.g. colored cells may indicate the cells expressing the exogenous genes). (3) Harvest and cultivation of the cells according to ES cell culture, e.g. using mitotically inactivated feeder cells. (4) Usually, a small subset of the transfected cells becomes iPS cells and generates ES-like colonies.

In the present invention the expression of Sox2 in the MAB-like cells is low as compared to embryonic stem cells. Accordingly, iPS may be produced by elevating the cells Sox-2 level, which may be achieved by
a) transfecting the mesoangioblast-like cell of the present invention with the Sox2 gene and expressing the same;
b) transducing the mesoangioblast-like cell of the present invention with the Sox2 protein; and/or
c) activating a promoter directing the Sox2 gene expression in the mesoangioblast-like cell of the present invention.

After some time (e.g. after 3-4 weeks), small numbers of transfected cells begin to become morphologically and biochemically similar to pluripotent stem cells, and are typically isolated through morphological selection, doubling time, or through a reporter gene and antibiotic infection.

Additionally, the MAB-like cells or iPSs may be genetically modified. This may be of particular interest in order to study the function and activity of a particular gene of interest (e.g. during embryonic development) or in order to produce animals with desired properties (e.g particular productive farm animals) resulting from the presence of a transgene or absence of a particular gene.

It is noted that also mesoderm-derived cells, as well as iPSs may be used as medicament as detailed for the MAB-like cells of the present invention.

The following figures and examples are offered by way of illustration and not by way of limitation.

### FIGURES

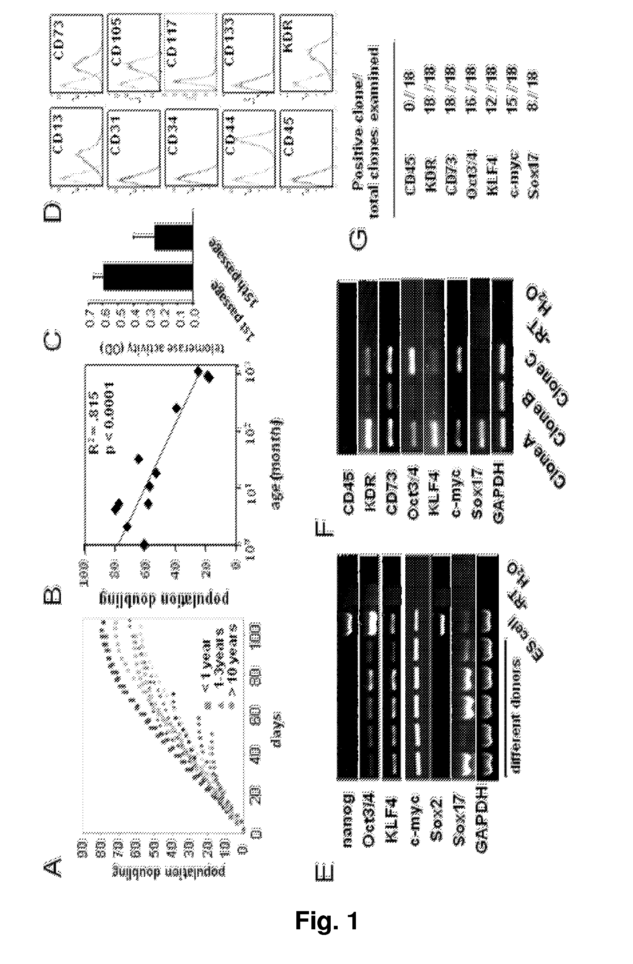

**Figure 1**(A) Population doubling of cells isolated of 12 different donors. Square : under 1 year old; triangle : 1-3 years; circle : over 10 years old. (B) Single regression analysis between age and population doublings. (C) Telomerase activity of 1^{st} and 15^{th} passage. n=11 (1^{st} passage), n=4 (15^{st} passage). (D) Flow cytometry analysis of child-derived cells. Isotype IgG served as control. Mesenchymal markers, CD13, CD73, CD44 are positive. Hematopoietic markers, CD34, CD45, CD117, and CD133 are negative. Some endothelial markers, such as KDR, CD105 are positive. (E) RT-PCR of stem cell markers. Cells were isolated from 5 different donors. Mouse embryonic stem cells (ES cells) are used as positive control. Samples without reverse transcriptase (-RT) and H₂O served as negative control. (F) Expression of markers in representative single cell-derived clones. (G) Number of single cell-derived clones expressing the respective marker.

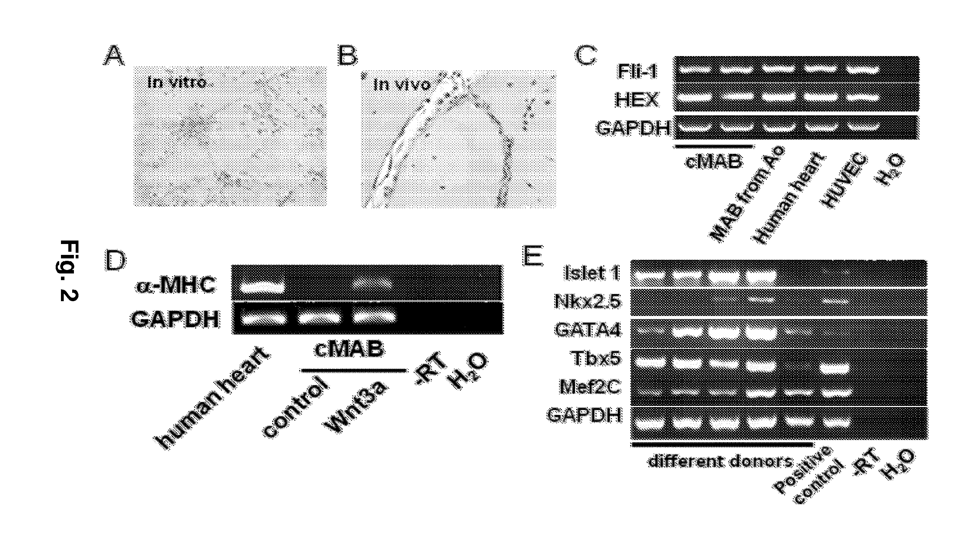

**Figure 2**(A-B) Endothelial differentiation in vitro (A; morphology) and in vivo (B; Hematoxilin Eosin staining) using matrigel plug assays. (C) RT-PCR of Fli-1 and HEX in peripheral blood-derived circulating mesoagioblasts (cMAB) isolated from 2 children, MAB obtained from human aorta, human heart, and HUVEC are shown. H₂O served as negative control. (D) RT-PCR of α-myosin heavy chain (α -MHC) and GAPDH of cMAB. Wnt3a and dexamethasone was used to induce cardiac differentiation. (E) RT-PCR of several transcription factors of cells isolated of 5 different donor-derived cMAB is shown. Human or rat embryonic heart are used as positive control. -RT and H₂O served as negative control.

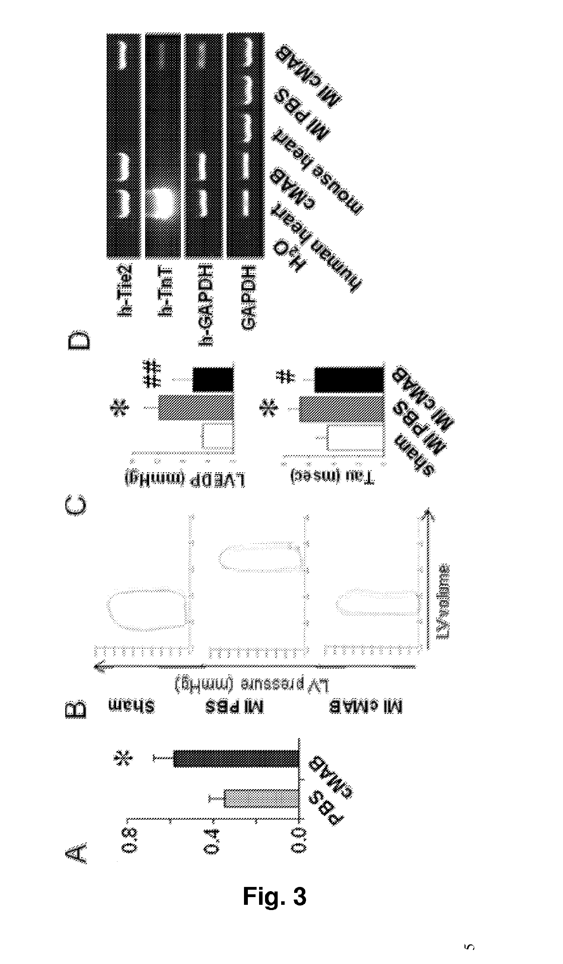

**Figure 3** Cell mediated functional recovery in a hind limb ischemia (A), and myocardial infarction (MI) model (B-D) using nude mice. (A) 1x10⁶ of children-derived cMABs were injected intramuscularly after ischemia and function was analyzed 2 weeks after operation. Summary of Doppler flow data in cMAB or PBS control group. n=4 (PBS) and 7 (cMAB). * p<0.05. (B-C) Pressure-volume loop analysis using Millar catheter was performed. Representative pressure-volume curve (B) and quantification (C) are shown. * indicates p< 0.01 vs sham. # and ## indicates p< 0.05 and p<0.01 vs MI PBS group, respectively. n=3 (sham), n=7 (PBS), and n=6 (cMAB). (D) Human specific RT-PCR of Tie2, troponin T, and GAPDH in heart injected with cMAB or PBS.

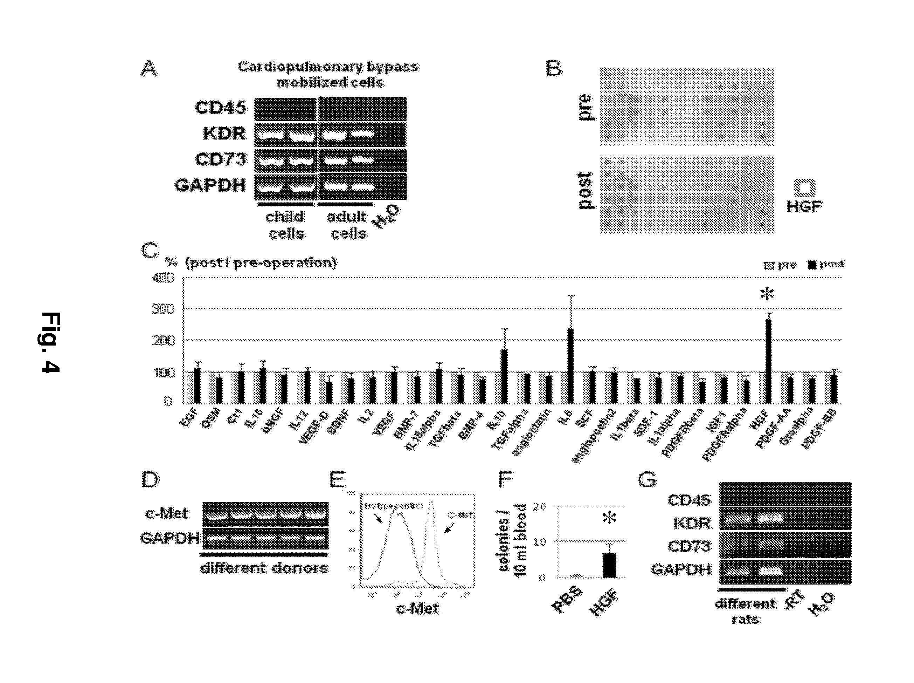

**Figure 4**(A) RT-PCR of CD45, KDR, and CD73 in cultivated cells, which were obtained from children or adults undergoing heart surgery with cardiopulmonary bypass. (B-C) Representative cytokine arrays (B) and quantification (n=4) (C) are shown. Serum levels of pre-operation (pre) and post-operation (post) are examined. Gray square indicates hepatocyte growth factor (HGF). * p< 0.05 vs pre-operation. (D) RT-PCR of the HGF receptor c-Met and GAPDH in children-derived cells from 5 different donors. (E) c-Met expression by FACS. (F) Colony number derived from mononuclear cells per 10 ml rat blood after 2 weeks in culture. HGF (1µg/kg; n=5) or PBS (n=14) was administered in rats intravenously and mononuclear cells were obtained after 24 hours. (G) RT-PCR of rat CD45, KDR, and CD73. Cells were obtained from rats after injection of HGF.

### EXAMPLES

### Methods

*Cell isolation & culture from human peripheral blood:* The ethics review boards of the universities Giessen and Marburg, and Frankfurt approved the protocol, and the study was conducted in accordance with the Declaration of Helsinki. Written informed consent was obtained from each patient or patient's parent. Blood samples were collected from the patients who underwent open heart surgery with cardiopulmonary bypass. Patients with known genetic disorder such as troponin mutation, Down syndrome, or CATCH22 were excluded. Total mononuclear cells (MNCs) were isolated by Ficoll density gradient centrifugation with Biocoll separating solution (Biochrom AG, Berlin, Germany). MNCs were plated at 8×10⁶ cells/ml on a fibronectin-coated dish in endothelial basal medium (EBM) with supplements (1 µg/ml hydrocortisone, 3-12 µg/ml bovine brain extract, 30-50 µg/ml gentamicin, 50 µg/ml amphotericin B, 10 µg/ml EGF, and 20% fetal calf serum). After 7 days in culture, non-adherent cells were discarded and cells were cultured for additional 7 days in same medium. On day 15, cells were detached by 0.25% Trypsin-EDTA (GIBCO) and were seeded at 5×10⁴ cells/ml on fibronectin-coated dishes. When cells reached 80% confluency, cells were subsequently passaged at 5×10⁴ cells/ml and were used for following analyses. Adult cells were also obtained from patients undergoing open heart surgery with cardiopulmonary bypass (n=5). In order to compare serum levels and cells before and after cardiopulmonary bypass, we collected 2ml blood from children before heart operation. Adult blood from healthy volunteers (n=5) was used for the control experiments.

Alternatively, X-vivo medium + autologous serum can be used. Mononuclear cells were suspended in X vivo-15 medium (Biowhittaker) supplemented with 1 ng/mL carrier-free human recombinant VEGF (R&D), 0.1 µmol/L atorvastatin (provided by Pfizer), and 20% human serum drawn from each individual patient. Cells were seeded at a density of 6.4x10⁵ cells/mm² at fibronectin-coated dishes (Roche).

*Single cell cloning:* For single cell cloning, cells of 2^{nd}-3^{rd} passage were labelled with CM-Dil (Invitrogen) and seeded at a single cell density on fibronectin-coated 96well plates. Single-cell deposition was confirmed microscopically and wells containing more than one cell were excluded.

*Other cells:* Human umbilical vein endothelial cells (HUVEC) were purchased from Cambrex and cultured in endothelial basal medium supplemented with 1 µg/mL hydrocortisone, 12 µg/mL bovine brain extract, 50 µg/mL gentamicin, 50 ng/mL amphotericin-B, 10 ng/mL epidermal growth factor and 10% fetal bovine serum. Mesenchymal stem cells (MSCs) were isolated by density-gradient centrifugation with Ficoll from bone marrow of healthy human volunteers. Immediately after isolation, cells were maintained in Mesencult medium (StemCell Technologies, Germany). The CD34⁺ hematopoietic progenitor cells were isolated from human peripheral blood by immunomagnetic purification. Human mesoangioblasts were obtained from the tissue of ascending aorta from explanted heart in collaboration with Dr. G. Cossu (A. Dellavalle et al., Nat Cell Biol 9, 255-67 (2007)). Aortic tissue was explanted on gelatin-coated dishes and outgrowth cells were used for the experiments.

*Hind limb ischemia, cell injection and functional evaluation:* The *in vivo* angiogenic capacity was examined in a unilateral hind limb ischemia model using 8- to 10-week-old athymic NMRI nude mice (Harlan, Borchen, Germany). The proximal portion of the right femoral artery including the superficial and the deep branch and the distal portion of the saphenous artery were occluded with an electrical coagulator. Then 1×10⁶ cells in 50 µl PBS were injected intramuscularly at 4 different sites. The overlying skin was closed by using surgical staples. After 2 weeks, we determined the ischemic (right)/nonischemic (left) limb blood flow ratio by using a laser Doppler blood flow imager (Laser Doppler Perfusion Imager System, moorLDI-Mark 2; Moor Instruments, Wilmington, DE). Data are expressed as the ratio of ischemic to nonischemic hind limb.

*Myocardial infarction, cell injection and functional evaluation:* Myocardial infarction was induced by permanent ligation of the left coronary artery in 10- to 12-week-old athymic NMRI nude mice (Harlan). Soon after ligation, 1×10⁶ cells or PBS (both 50µl) were injected intramuscularly into the border zone at 3 different sites. On day 14, cardiac catheterization was performed for functional analysis by using 1.4F micromanometer-tipped conductance catheter (Millar Instruments Inc). Left ventricular (LV) pressure and its derivative (LV dP/dt) were continuously monitored with a multiple recording system. All data were acquired under stable hemodynamic conditions.

*Mobilization of mesoangioblasts-like cells into circulation:* We intravenously injected 1µg/kg recombinant human hepatic growth factor (rhHGF) or PBS into Lewis Rats. We collected 2-8 ml peripheral blood (PB) from each rat. Cell isolation and culture was performed as described for human cells.

*Flow cytometry:* Following antibodies (Abs) were used for fluorescence activated cell sorting (FACS): Phycoerythrin (PE)-conjugated anti-CD13, CD14, CD31, CD34, CD44, CD45, CD73, CD146 (BD Biosciences), CD 105, CD144, KDR (R&D), CD133 (Miltenyl Biotec), Fluorescein (FITC)-conjugated anti-CD117 (Santa Cruz), biotinylated c-Met, Allophycocyanin (APC)-conjugated streptavidin, and isotype-matched PE, FITC, or APC-conjugated mouse immunoglobulins. Samples were analyzed by a flow cytometer, BD FACS Calibur cell sorter (BD Biosciences, San Jose, CA).

*RT-PCR:* Total RNAs were isolated by using TRIzol (Invitrogen) or RNeasy Mini Kit (Qiagen). RNA was subjected to RT-PCR by using SuperScript First Strand Synthesis System (Invitrogen). The primer sequences are available upon request. For subcloning and sequencing RT-PCR products were purified by Gel Extraction kit (Qiagen) and were subcloned by using pGEM-T Easy vector (Promega) and 6 clones were collected. Each clone was amplified and sequences were analyzed (SeqLab, Germany).

*Chromatin immunoprecuipitation (ChIP) Assay:* For each immunoprecipitation, approximately 5x10⁶ cells were crosslinked with 1% formaldehyde for 5 min. at room temperature and quenched by addition of glycine (1.25M). The cell lysate was sonicated by using a Branson 450 Sonifier with 4 pulses for 5 sec. with 30% Output to sheer chromatin-DNA complex. For histone ChIPs, EZ ChIP kit (Upstate Biotechnology) was used and followed the manufacturer's protocols. In this assay, 5 µg of AcetylH3 (06-599 Upstate), H3K9me3 (07-442 Upstate), H3K27me3 (07-449 Upstate), H3K4me3 (07-473 Upstate), and 1µg of IgG(13-371B Upstate), were used. The total input or immunoprecipitated DNA was determined by quantitative RT-PCR with the equation. ΔCt (threshold cycle) of each sample = mean of Ct_{(antibody)} - mean of Ct₍ᵢₙₚᵤₜ₎. Primers are listed below.

*Telomerase activity:* Telomerase activity was measured with a Telomerase ELISA Assay Kit (Millipore) according to the manufacturer's instructions. 1x 10⁶ cells were used for each experiment.

*In vitro Osteogenic, smooth muscle, and cardiac differentiation assay in vitro:* Several differentiation assays were performed as summarized in Table 1.

**Table 1: In vitro differentiation assay of children derived circulating mesoangioblasts-like cells for osteoblasts, smooth muscle cells (SMC), and cardiomyocytes (CM). (Stimuli, matrixes, medium, and duration of the cultivation are indicated)**

| | **stimuli** | **matrix** | **medium** | **duration** |
|---|---|---|---|---|
| osteoblasts | BMP2 (100 ng/ml) | fibronectin | DMEM with 10% FBS | 8 days |
| smooth muscle differentiation condition 1 | TGFb1 (5 ng/ml) | fibronectin | DMEM with 10% FBS | 14 days |
| smooth muscle differentiation condition 2 | FGF8b (30 ng/ml) Heparin (10 mg/ml) | Jagged-1 (10mg/ml) | DMEM + medium 199 (4:1) with 8% FBS | 3, 7 and 14 days |
| cardiac myocytes differentiation condition 1 | Co-culture with rat CM | gelatine | DMEM + medium 199 (4:1) with 8% FBS | 6 days |
| cardiac myocytes differentiation condition 2 | Wnt3a (100 ng / ml) Dexamethasone (10 nM) | none | DMEM / F-12 (Ham) (1:1) with 10% FBS | 3 days |

Alkaline phosphatase (ALP) expression was confirmed by StemTAG™ Alkaline Phosphatase Activity Assay Kit (Cell Biolabs Inc.) according to the manufacturer's instructions. Immunofluorescence was performed to confirm smooth muscle cells (SMC) differentiation using anti-smooth muscle actin (SMA) antibody after 14 days of culture. To induce cardiac differentiation, cells were co-cultured with neonatal cardiomyocytes (CM) for 6 days as previously described with a minor change (C. Badorff et al., Circulation 107, 1024-32 (2003); M. Koyanagi et al., J Biol Chem 280, 16838-42 (2005)). In brief, cells were added onto CM at a ratio of 1:6 two days after CM isolation. After 6 days, cells were used for detection of cardiac marker gene expression. Alternatively, cells were cultured on non-coated dishes and Wnt3a (100 ng/ml) and dexamethasone (10nM) were added without co-culturing rat CM.

*Tube Formation Assay (In Vitro Matrigel Plug Assay):* Cells (2×10⁵) were cultured in a 12-well plate (Greiner) coated with 200 µL of Matrigel Basement Membrane Matrix (BD Biosciences). Tube length was quantified after 48 hours by measuring the cumulative tube length in four random microscopic fields with a computer-assisted microscope using the program KS300 3.0 (Zeiss).

*In Vivo Matrigel Plug Assay:* This assay was performed as described previously (A. Kuehbacher, C. Urbich, A. M. Zeiher, S. Dimmeler, Circ Res 101, 59-68 (2007)). Briefly, CM-Dil (Invitrogen) labeled cells (1×10⁶) were resuspended in 30 µl PBS and mixed with 500 µl of Matrigel Basement Membrane Matrix (BD Biosciences) containing 15 U of heparin (Sigma-Aldrich). The cell-matrigel mixture was injected subcutaneously into 6- to 8-week-old female athymic nude mice (Harlan) along the abdominal midline. After 7 days, blood vessel growth in Matrigel plugs was quantified by analysis of CD31-stained sections using microscopy. For hemoglobin analysis, the Matrigel plug was removed after 7 days and homogenized in 130 µL of deionized water. After centrifugation, the supernatant was used in the Drabkin assay (Sigma-Aldrich) to measure hemoglobin concentration. Stock solutions of hemoglobin are used to generate a standard curve. Results are expressed relative to total protein.

*Histological analysis:* For morphological examination, the hearts of the mice and Matrigelplugs were fixed with 10% buffered formalin and embedded in paraffin, processed for light microscopy, and stained with hematoxylin and eosin. Alternatively, the hearts of mice and Matrigelplugs were embedded in optimal cutting temperature compound (O.C.T. compound), and were quickly frozen in liquid nitrogen and cut in 5 µm sections.

For immunohistochemistry of cultured cells, cells were fixed with 4% paraformaldehyde. After permeabilization with 0.2% saponin (Sigma), cells were incubated with the respective antibodies (CD31; Chemicon, smooth muscle actin; Sigma, alpha sarcomeric actinin; Sigma, GATA4; Santa Cruz, Nkx2.5; Santa Cruz). The specimens from frozen tissue sections were fixed with 4% paraformaldehyde, followed by staining with the respective antibodies (human nuclear antigen; Chemicon, smooth muscle actin; Sigma, alpha sarcomeric actinin; Sigma). Nuclei were counterstained with To-pro-3 iodide, Sytox Blue (both Molecular Probes) or DAPI according to the manufacturer's instructions. The images were recorded by confocal microscope (LSM510-META, Carl Zeiss, Oberkochen, Germany).

For fluorescence in situ hybridization 6 µm sections were prepared from formalin-fixed, paraffin-embedded tissue blocks according to standard procedures. After deparaffinization sections were submitted to heat-induced epitope retrieval by boiling for 22 minutes in 1 mmol/L sodium citrate buffer (pH 8.0). Sections were fixed with 1% paraformaldehyde/PBS on ice for 10 minutes. The human Alu probes were used for fluorescence in situ hybridization (FISH) (C. Bearzi et al., Proc Natl Acad Sci U S A 104, 14068-73 (2007)). Probes were dehybridisized for 4 min. at 71°C before incubation of probes with samples (4 min. at 80°C). Overnight hybridization at 37°C was followed by a stringent wash (2× SSC with 50% formamide 2 times for 10 minutes, 2 x SSC for 5 minutes, NP-40 /2 x SSC for 5 minutes, 2 x SSC for 5 minutes at 42°C). Sections were blocked for 30 minutes with bovine serum albumine and incubated with antibodies against smooth muscle actin (mouse IgG, 1:400, DAKO) for 1 hour. After washing cells were incubated with Alex Fluor 647-conjugated secondary antibodies (1:200) (donkey antimouse IgG, Molecular Probe). Nuclei were stained with DAPI (DAPI Mounting medium, Vector Laboratories) or Cytox blue (1:2000, Molecular Probes). All staining were detected using confocal microscopy (Zeiss LSM510 system, Germany).

*Cytokine Array:* Patient's serum was analyzed by using a cytokine antibody array from RayBio Human Cytokine Antibody Array (RayBiotech, Inc, Norcross, Ga) according to the manufacturer's instructions. A semiquantitative analysis of the comparative intensity of the spots was performed with an image analysis program (TINA; version 2.09g).

*Primer list:*

| **PRIMER NAME** | **PRIMER SEQUENCE** | **SEQ ID NO:** |
|---|---|---|
| h-GAPDH-for | GAAGGTGAAGGTCGGAGTC | 1 |
| h-GAPDH-rev | GAAGATGGTGATGGGATTTC | 2 |
| hmr-GAPDH-for | CAGAAGACTGTGGATGGCCC | 3 |
| hmr-GAPDH-rev | AGTGTAGCCCAGGATGCCCT | 4 |
| hm-Nanog-for | CAGCCCTGATTCTTCCACCA | 5 |
| hm-Nanog-rev | GTTCTTGCATCTGCTGGAGG | 6 |
| hm-Oct3/4-for | GAACATGTGTAAGCTGCGGC | 7 |
| hm-Oct3/4-rev | TTCTGGCGCCGGTTACAGAA | 8 |
| hm-KLF4-for | CCCACATTAATGAGGCAGCC | 9 |
| hm-KLF4-rev | TGGGGGAAGTCGCTTCATGT | 10 |
| hm-cMyc-for | AACGACAGCAGCTCGCCCAA | 11 |
| hm-cMyc-rev | TTGAGGACCAGTGGGCTGTG | 12 |
| hm-SOX17-for | ATACGCCAGTGACGACCAGA | 13 |
| hm-SOX17-rev | TAGTTGGGGTGGTCCTGCAT | 14 |
| hm-Sox2 for | AGATGCACAACTCGGAGATCAGC | 15 |
| hm-Sox2 rev | GCCGTTCATGTGCGCGTAACTGTC | 16 |
| hm-CD45 for | CCAGGAAATACATTGCTGCACAAG | 17 |
| hm-CD45 rev | TCGCCTTAGCTTGACAACATAACC | 18 |
| h-KDR-for | CTCTGCCAATCCATGTGGGA | 19 |
| h-KDR-rev | CGTGCATGAGACTTCGATGC | 20 |
| CD73 for | TGATCGAGCCACTCCTCAAAGAG | 21 |
| CD73 rev | CTCTTTGGAAGGTGGATTGCCTG | 22 |
| h-Fli1-for | ATCAGCCAGTGAGGGTCAAC | 23 |
| h-Fli1-rev | GGCCATTCTTCTCGTCCATA | 24 |
| hm-HHEX-for | ACCATCGAGCTGGAGAAGAA | 25 |
| hm-HHEX-rev | TGCTTTGAGGGTTCTCCTGT | 26 |
| hTropT Exon9 for | GAAGAAGAAGAGGAAGCAAAGGAG | 27 |
| hTropT Exon9 rev | TCCTTCTCCCGCTCATTCC | 28 |
| Islet-1-for | AGTGTAATCAGTATTTGGACGAGAG | 29 |
| Islet-1-rev | TAGCAGGTCCGCAAGGTGTGCAG | 30 |
| h-Nkx2.5-for | CACCGGCCAAGTGTGCGTCT | 31 |
| h-Nkx2.5-rev | CCGCGTTGTCCGCCTCTG | 32 |
| h-GATA4-for | GACGGGTCACTATCTGTGC | 33 |
| h-GATA4-rev | CACTACCTGAAGGAGCTGC | 34 |
| MK-hTbx5-for | CCACAGCTGGGAGAGGGAAT | 35 |
| MK-hTbx5-rev | CAACTCCGTGCACAGAGTGG | 36 |
| MK-hMEF2C-for | CATCTGGTTGGTTTTCAGCC | 37 |
| MK-hMEF2C-rev | CGGATGACAAAGCAACTATCC | 38 |
| h-Tie2-for | AGCTGCAACAATGGGGAGAT | 39 |
| h-Tie2-rev | TGTTGCCAAGCCTCATAGTG | 40 |
| h-a-MHC-for | GTCCCGGCAGCTAGAGGA | 41 |
| h-a-MHC-rev | CCTCTGTCTCCTCCTCGTAC | 42 |
| r-CD45-for | CCACCAGGGACTCACAACTT | 43 |
| r-CD45-rev | GTAGAGGACTTCCGCAGCAC | 44 |
| r-CD73-for | ATGCCTTTGGCAAATACCTG | 45 |
| r-CD73-rev | GGTTTCCCATGTTGCACTCT | 46 |
| r-KDR-for | CCAAGCTCAGCACACAAAAA | 47 |
| r-KDR-rev | CCAACCACTCTGGGAACTGT | 48 |
| rGAP-for | TTGTGATGGGTGTGAACCAC | 49 |
| rGAP-rev | GGATGCAGGGATGATGTTCT | 50 |
| h-CD34-for | CACCCTGTGTCTCAACATGG | 51 |
| h-CD34-rev | GGCTTCAAGGTTGTCTCTGG | 52 |
| CS-CD13 for | ACAGCCAGTATGAGATGGACAGC | 53 |
| CS-CD13 rev | CCATTGGATGCCTGCTTCTTCAC | 54 |
| h-CD14 for | GCCGCTGGTGCACGTCTCTGC | 55 |
| h-CD14 rev | GGCTTCCAGAGGCAGCGGAGG | 56 |
| CD18 for | GCCCCAACAAGGAGAAAGAGTGC | 57 |
| CD18 rev | CGATGGGTAGTCGAATTCGTTGC | 58 |
| CD29 for | CCTTACATTAGCACAACACCAGC | 59 |
| CD29 rev | TTTTTCAGCTCCTTGTAAACAGGC | 60 |
| CD31 for | GGATCATTTCTGGGATCCATATGC | 61 |
| CD31 rev | GCAGATATACGTCCCACTGTCCG | 62 |
| CD44 for | AGAAAGCTCTGAGCATCGGATTTG | 63 |
| CD44 rev | TGTAACCTCCTGAAGTGCTGCTC | 64 |
| CD105 for | CATCGACGCCAACCACAACATGC | 65 |
| CD105 rev | TGCACTTCAAATGCGCAACAAGC | 66 |
| CD117 for | ATTCCCCAAACCTGAACACCAGC | 67 |
| CD117 rev | CAAAAATACCAATCTATTGTGGGC | 68 |
| CD 133 for | ATCAAAAGGAGTCGGAAACTGGC | 69 |
| CD133 rev | TTGGTGCAAGCTCTTCAAGGTGC | 70 |
| VE-Cad-for | AGGTATGAGATCGTGGTGGAAGC | 71 |
| VE-Cad-rev | TGGATGTATTCATAATCCAGAGGC | 72 |
| CD146 for | AAGATGCCCAGTTTTACTGTGAGC | 73 |
| CD146 rev | GTTCCTGTGGTTCACTCAGCAGC | 74 |
| h-NG2-CSPG4-for | TGCTGCAGCTCTACTCTGGA | 75 |
| h-NG2-CSPG4-rev | CTGAGGAGGCGTTCAGAAAC | 76 |
| c-met for | GCCTGAATGATGACATTCTTTTCG | 77 |
| c-met rev | GCGACCCTCTGATGTCCCAAGAT | 78 |
| ChIP KLF4 for | GCTGCTGAGTGGAAGAGAGC | 79 |
| ChIP KLF4 rev | AATTGGCCGAGATCCTTCTT | 80 |
| hOCT3/4-ChIP-S2 | TTGCCAGCCATTATCATTCA | 81 |
| hOCT3/4-ChIP-AS2 | TATAGAGCTGCTGCGGGATT | 82 |
| hSOX2-ChIP-S1 | GAGAAGGGCGTGAGAGAGTG | 83 |
| hSOX2-ChIP-AS1 | AAACAGCCAGTGCAGGAGTT | 84 |

| | | |
|---|---|---|
| SEQ ID NO: 81 and 83: cf. K. Takahashi et al., Cell 131, 861-72 (2007) | | |

*Statistics:* Data are expressed as mean ± SEM. Unpaired two-tailed student's t-test, Mann-Whitney U-test (to compare 2 groups) or ANOVA (≥ 3 groups) were used for the comparison between groups based on the original data. Single regression analysis was performed to investigate the relation of donor age and population doubling.

### EXAMPLE 1: Identification of MAB-like cells from peripheral blood of children

Circulating progenitor cells contribute to neovascularization and have been used for cell therapy of ischemia. Endothelial progenitor cells (EPC) isolated from adult blood are characterized by coexpression of hematopoietic and endothelial markers, analogue to the hemangioblast. In contrast, in embryonic development, vessel-associated multipotent progenitors, the so called mesoangioblasts (MAB), have been experimentally described. MAB express the key marker of angiopoietic progenitors, KDR, as well as mesenchymal markers.

In order to assess the phenotype and functional properties of circulating progenitors during postnatal development, we isolated cells from peripheral blood of children between 8 days and 2.5 years after birth and compared these cells with adult EPC. For this, isolated mononuclear cells were plated on fibronectin-coated dishes in EBM medium supplemented with growth factors.

At day 14, adherent cells derived from children demonstrated expression of mesenchymal markers CD13, CD73, the endothelial markers CD105, KDR and VE-cadherin, but were negative for hematopoietic markers CD133 and CD45 as documented by FACS analysis and RT-PCR (figure 1d/data not shown). In contrast, adult EPC did express hematopoietic and endothelial markers, but lacked expression of CD73 (table 2). Thus, the children-derived circulating progeniors (ChCP) resemble the previously described multipotent MAB. Indeed, ChCP could be induced to differentiate to cardiomyocytes, smooth muscle cells, and osteoblasts (figure 2/data not shown). Moreover, ChCP did express the stem cell markers islet-1 and c-kit and showed a marked proliferative capacity (28.3±0.9 passages, 64.0±2.9 population doublings) before entering a senescent state. Population doubling was strongly correlated with age of donors (R²=0.824, p=0.0001). Consistently, ChCP had a high telomerase activity (OD 0.6±0.1) and secreted high levels of cytokines such as HGF, VEGF, and SDF-1 (figure 1/data not shown). These data identify a novel circulating progenitor cells, which can be isolated during postnatal development in humans. These ChCP resemble the previously described multipotent MAB during embryonic development, exhibit a high proliferative capacity and express various functional stem cell markers. These progenitors might be well suited for cell therapeutic strategies.

### EXAMPLE 2: Circulating mesoangioblasts differentiate into cardiac myocytes and improve function after acute myocardial infarction

The cells described in Example 1 were tested for their potential of cardiac differentiation For this, we tested the capacity of children-derived MAB to acquire a cardiomyogenic phenotype.

MAB expressed several cardiac transcription factors such as Nkx2.5, GATA4 and MEF2C and the stem cell marker islet-1 (figure 2e). In order to assess cardiac differentiation capacity, we performed co-culture assays with neonatal rat cardiomyocytes (CM). Immunochemical analysis revealed that MAB expressed cardiac α-sarcomeric actinin 6 days after co-culture (data not shown). Moreover, human troponin T (TnT) was expressed as demonstrated by human specific RT-PCR (data not shown). To confirm these data, we examined TnT expression in MAB isolated of a 2-year old patient with a known mutation of TnT. Sequences of the cloned RT-PCR products were identical to human TnT except for the known mutation providing genetic proof of concept for cardiac differentiation. In order to exclude fusion between MAB and CM as a mechanism, we used paraformaldehyde-fixed CM as scaffold. In this assay, human TnT also was detected, indicating that differentiation is sufficient to induce cardiac markers gene expression. Next, we tested the effect of MAB to improve cardiac function. MAB was injected intramuscularly in nude mice after myocardial infarction. Functional analysis using Millar catheter 2 weeks after infarction demonstrated that cell therapy lowered filling pressure and preserved diastolic function when compared with PBS injected group (LVEDP: - 20.3%, tau: -20.6%, vs PBS injected heart). Furthermore, left ventricular volume was also decreased (LVEDV/weight -27.3%) (figure 3).

In summary, children-derived MAB express cardiac-specific genes after co-culture with CM and improved cardiac function *in vivo.* Given that MAB can be easily isolated and expanded from peripheral blood, these cells might be suitable to augment cardiac repair in children with heart failure.

### EXAMPLE 3: Adult circulating progenitor cells for cardiovascular regeneration

Cell therapy is an option to improve cardiac function of heart failure. Several types of cells were reported to induce angiogenesis and cardiogenesis *in vitro* and *in vivo.* Circulating blood is an attractive and clinically feasible source to isolate stem or progenitor cells. Endothelial progenitor cells (EPC), which is one population of circulating progenitors, can differentiate to endothelial phenotype and can release several growth factors, which contributed to neovascularization in ischemic tissue. Indeed, several clinical trials showed beneficial effect of EPC on improvement of ischemia and cardiac function. Though cardiac differentiation of EPC was shown *in vitro* and *in vivo,* the rate was too low to explain the beneficial effects of cell therapy. Therefore, the elucidation of the mechanism, by which the expression of cardiac genes is induced, may help to design strategies to further enhance the repair capacity of the cells.

*In vitro* studies established that the presence of cardiomyocytes was required to induce cardiac differentiation of EPC suggesting that cell-to-cell contact would be indispensable for this process. Notch families of proteins are transmembrane receptors. After binding with their ligands, Notch intracellular domain (NICD) is cleaved and contributes to cardiovascular development. Notch activation was transiently detected in EPC as determined by immunhistochemical detection of the NICD and expression of human Notch target genes. Inhibition of γ-secretase blocked Notch cleavage and NICD translocation. Furthermore, the expression of the cardiac marker proteins were significantly suppressed by γ-secretase inhibition indicating that Notch activation facilitates cardiac marker gene expression. Because non-canonical Wnts have previously been shown to promote cardiac differentiation, we additionally determined the influence of Notch activation on the expression of Wnt5a. Wnt5a expression was induced in the human cells by the co-culture and was blocked by γ-secretase inhibitor. Likewise, stimulation of Notch signaling by immobilized Jagged-1 promoted Wnt5a expression in EPC. These data indicated that Notch and Wnts signaling contributed to cardiac differentiation of EPC. Importantly, Wnt5a pretreated EPC can differentiate to CM in vivo after myocardial infarction, suggesting this enhancing strategy might be suitable for cardiac repair.

However, the number and the functional capacity of EPC are impaired in patients with coronary artery disease. In contrast to adult cells, child cells might have better cell function. Therefore, we compared circulating progenitor cells isolated from children and from adults, and found that progenitor cells from children have a greater proliferative capacity which was significantly correlated with age. These cells expressed mesenchymal markers, and negative for hematopoietic marker CD45, have high telomerase activity and are positive for several stem cell markers (islet-1, and Oct3/4) (figure 1e). The marker profiles of the clonally expanded cells is distinct from adult circulating "endothelial" progenitor cells (EPC), but resemble but are not identical as mesoangioblasts and unrestricted somatic stem cells (USSCs).

Since child progenitor cells additionally expressed several cardiac transcription factors (GATA4 and Nkx2.5), we investigated the capacity to differentiate into cardiomyocytes. After co-culture with neonatal rat cardiomyocytes, children-deribed progenitor cells expressed cardiac α-sarcomeric actinin. Moreover, co-culture of child progenitor cells with rat cardiomyocytes led to a higher expression of human troponin T, as assessed by human specific RT-PCR, as compared to EPC.

Furthermore, the intramyocardial injection of these progenitor cells into nude mice following myocardial infarction led to a significant functional improvement (LVEDP: - 20.3%, tau: -20.6%, LVEDV/weight -27.3%, vs control).

In summary, novel progenitor cells isolated from children have a greater proliferative and greater cardiomyocyte differentiation capacity than EPC, and improve cardiac function after myocardial infarction. Therefore, these cells may be an alternative for the heart failure treatment.

### EXAMPLE 4: Identification of a novel subset of multipotent circulating stem cells with a high capacity for cardiovascular regeneration

In Example 1 we identified circulating MAB-like cells in children. Children-derived MAB like cells (cMABs) showed vigorous proliferation capacity and high telomerase activity. Although, cMABs showed several stem cell features, the potential for cardiovascular repair and regeneration is not known.

Therefore, we assessed the lineage differentiation potential by culturing cMABs under conditions favouring endothelial cell (EC), smooth muscle cell (SMC) and cardiomyocyte (CM) differentiation. EC differentiation was determined by using matrigel assays (figure 2a/b). Tube like-structures were formed in vitro and in implanted matrigel plugs in vivo to a similar extent as compared to human umbilical cord endothelial cells (HUVEC). cMABs expressed endothelial markers and connected to the mouse vasculature leading to increased perfusion of the plugs (+133% increase). Consistent with the capacity of the cells to differentiate to the endothelial lineage, the transcription factors Hex and Fli-1 known to play a key role in vascular development were highly expressed (figure 2c). Differentiation into SMC was achieved in individual cells by addition of TGFβ1, while cultivation of the cells on jagged-coated dishes in combination with FGF8 induced the expression of SMC-specific proteins in > 60 % of all cells. In order to induce differentiation into the cardiac lineage, cMABs were co-cultured with neonatal rat CM. After 6 days of co-culture, cMABs acquired a CM fate as demonstrated by the expression of α-sarcomeric actinin by immunohistocemistry (IHC) and by the expression of human troponin T (TnT) mRNA. Even in the absence of co-culture, incubation with Wnt3a, which was previously shown to promote CM differentiation of embryonic stem cells, increased the expression of Nkx 2.5, α-myosin heavy chain, and TnT. In line with these results, cells expressed various muscle transcription factors such as GATA-4, Mef-2C, while only some clones expressed Nkx2.5. Interestingly, cMABs strongly expressed Islet-1, which was previously shown to make a multipotent primordial cardiovascular progenitors. Taken together, cMABs are multipotent and can differentiate into the 3 distinct cardiovascular cell lineages.

In order to determine the potential functional benefit using cMABs for therapeutic applications, cells were injected in a nude mice model of hind limb ischemia. After 14 days, the recovery of blood flow was significantly greater in mice treated with cMABs compared to PBS-treated control mice. In addition, cMABs were injected in mice after induction of myocardial infarction. After 2 weeks, cell-treated mice exhibited a smaller infarct size and a significantly improved cardiac function with lower left ventricular end-diastolic pressures, and improved diastolic function (Tau) (LVEDP: -56.0%, Tau: -20.3%). The in vivo differentiation of the injected cells to all three cardiovascular lineages was assessed by IHC.

In summary, cMABs differentiated to all three cardiovascular lineages in vitro and in vivo. Moreover, cMABs improved neovascularization and cardiac function after ischemia. Given that these circulating cMABs can be easily isolated and expanded from peripheral blood, these cells might be suitable to augment cardiac repair in children with heart failure.

### EXAMPLE 5: Detailed characterization of circulating mobilizable multipotent mesoangioblasts in humans

Blood-derived circulating mononuclear cells were obtained from children undergoing cardiopulmonary bypass for cardiac surgery. Cells were cultivated on fibronectin-coated dishes and first clones were detected after 1-2 weeks. Cells showed a spindle-shaped morphology , exhibited a high proliferative capacity, and were cultured for 28.3±0.9 passages (Fig. 1A). The proliferative capacity was closely correlated with the donor age (Fig. 1A/B) and was associated with a high telomerase activity, which was detected at least until the 15^{th} passage (Fig. 1 C). Onset of senescence started after the 20^{th} passage (0.25 %) and approximately 10 % acidic β-galactosidase-positive cells were detected after 29 passages. Characterisation of the surface marker expression by flow cytometry and RT-PCR revealed that the cultivated cells express the mesenchymal markers CD13, CD73, and CD44, the endothelial markers CD105 and KDR, but were negative for the endothelial/hematopoietic marker CD31 and the hematopoietic markers CD34, CD133, and CD45 (Fig. 1D and data not shown)). Table 2 provides a summary of the expression of various markers by RT-PCR and flow cytometry of the cultivated cells compared to other cells.

**Table 2: Marker expressions (summary of FACS and RT-PCR)**

| **CD** | **HUVEC** | **MSC** | **CD34+ cells** | **MAB** | **Children-derived MAB-like cells** | **Umbilical cord- blood-derived cells** |
|---|---|---|---|---|---|---|
| **Endothelial marker** | | | | | | |
| CD31 | +++ | - | ++ | - | - | + |
| CD34 | + | - | +++ | not performed | - | not performed |
| CD105 | +++ | +++ | ++ | +++ | + to +++ (heterogeneous) | + |
| CD144 | +++ | - | + | - | + | + |
| CD309 (KDR) | +++ | - | + | + | +++ | + |
| **Mesenchymal marker** | | | | | | |
| CD13 | +++ | +++ | +++ | +++ | +++ | + |
| CD73 | +++ | +++ | - | +++ | +++ | - |
| Hematopoietic marker | | | | | | |
| CD45 | - | - | +++ | - | - | +++ |
| CD133 | - | - | +++ | - | - | |
| **Adhesion / Integrin marker** | | | | | | |
| CD18 | - | - | not performed | - | - | +++ |
| CD29 | +++ | +++ | not performed | +++ | +++ | +++ |
| CD44 | + | +++ | +++ | +++ | +++ | +++ |

| | | | | | | |
|---|---|---|---|---|---|---|
| Marker profiles of human umbilical vein endothelial cells (HUVEC), mesoangioblasts (MAB) from human aorta, CD34 positive cells, mesenchymal stem cells (MSC), children-derived MAB-like cells, and umbilical cord blood-derived cells are shown. Confirmed data by both FACS and RT-PCR are summarized. - , +, ++, +++ indicate no expression (0%), low expression (1-40%), high expression (40-80%), and highly expression (80-100%) by FACS measurement, respectively. Umbilical cord blood-derived cells were obtained from umbilical cord blood by using same protocol as children-derived MAB-like cells. | | | | | | |

The high expression of KDR clearly distinguishes the isolated cells from mesenchymal stem cells (Table 2). Moreover, although the cells are CD 105⁺KDR⁺, the absence of CD31 and lack of the hematopoietic markers CD34 and CD45 distinguish the cultivated children-derived cells from circulating endothelial and hematopoietic progenitor cells (Table 2 and (Ingram et al., Blood 104. 2752-60 (2004), Lin et al, J Clin Invest 105, 71-7 (2000), Timmermans et al., Arterioscler Thromb Vasc Biol 27, 1572-9 (2007)). When comparing the marker expression profile to previously described cells, these children-derived cells resemble mesoangioblasts, which are multipotent progenitors of mesodermal tissue originally isolated from the embryonic dorsal aorta and characterised by the expression of mesenchymal and endothelial markers (Cossu and Bianco, Curr Opin Genet Dev 13, 537-42 (2003)). Consistent with the phenotype of mesoangioblasts isolated from adult tissue (Dellavalle et al., Nat Cell Biol 9, 255-67 (2007)), children-derived cells express the proteoglycan NG2, a marker for pericyte-derived cells.

Next, we characterized the expression of stem cell markers. Among the four factors Oct3/4, Klf4, c-myc and Sox2, which are sufficient to induce pluripotency in human fibroblasts (Takahashi et al., Cell 131, 861-72 (2007)), the children-derived mesoangioblast-like cells expressed Oct3/4, Klf4 and c-myc, while Sox2 expression was below the detection limit (Fig. 1E/F). In accordance, chromatin immunoprecipitation demonstrated that histone modifications associated with active transcription (histone H3 acetylation and H3 lysine 4 trimethylation) were present at the Oct3/4 and Klf4 promotor region, while heterochromatin modifications (trimethylated H3 lysine 9 and trimethylated H3 lysine 27) were low or absent . In contrast, the repressive histone modifications trimethyl-H3 lysine 27 was high at the Sox2 promotor (Fig. 1G). The stem cell marker Nanog (Chambers et al., Cell 113, 643-55 (2003), K. Mitsui et al., Cell 113, 631-42 (2003)) was not detected (Fig. 1E), whereas Sox17, which was shown to be specifically expressed in fetal and neonatal hematopoietic stem cells (Kim, T. L. Saunders, S. J. Morrison, Cell 130, 470-83 (2007), was abundantly expressed (Fig. 1E).

To elucidate whether the cells can be cloned on a single cell level, we performed limited dilution assays using cells from the 2^{nd} -3^{rd} passage. The clonal efficiency estimated by single cell deposition was 9.5±2.0 %. 18 expanded single cell-derived clones were further characterised and all clones showed the characteristic expression of CD73 and KDR, but lacked expression of CD45 (Fig 1F/G). The expression of stem cell markers like Oct3/4, Klf4 and Sox 17 was more heterogeneous (Fig 1F/G). Collectively, these data indicate that in children undergoing cardiopulmonary bypass, the blood contains circulating mesoangioblast-like cells, which can be clonally expanded and express a variety of classical stem cell markers.

Next, we assessed the lineage-directed differentiation potential by culturing the isolated cells under conditions favoring osteogenic, endothelial, smooth muscle and cardiomyocyte differentiation. After 2 weeks, osteogenic differentiation was detected, albeit at low frequency. Endothelial differentiation was determined by using matrigel assays. Tube like-structures were formed in vitro and in implanted matrigel plugs in vivo (Fig. 2B) to a similar extent as compared to human umbilical cord endothelial cells (HUVEC). Implanted cells expressed endothelial markersand connected to the mouse vasculature leading to increased perfusion of the plugs. Consistent with the capacity of the cells to differentiate to the endothelial lineage, the transcription factors Hex and Fli-1, known to play a key role in vascular development (Guo et al., Blood 102, 2428-35 (2003), Hart et al., Immunity 13, 167-77 (2000)), were highly expressed (Fig. 2C). Differentiation into smooth muscle cells was achieved in individual cells by addition of TGFβ1, while cultivation of the cells on jagged-coated dishes in combination with FGF8 induced the expression of smooth muscle-specific proteins in > 60 % of all cells. In order to induce differentiation into the cardiac lineage, cells were co-cultured with neonatal rat cardiomyocytes (Laugwitz et al., Nature 433, 647-53 (2005), Badorff et al., Circulation 107, 1024-32 (2003)). After 6 days of co-culture, children-derived cells acquired a cardiomyocyte fate as demonstrated by the expression of α-sarcomeric actinin by immunostaining, and by the expression of human troponin T mRNA. In order to exclude fusion, we co-cultured cells with paraformaldehyde-fixed cardiomyocytes. Again, human troponin T mRNA was detected (data not shown). Even in the absence of co-culture, incubation with Wnt3a, which was previously shown to promote cardiac differentiation of embryonic stem cells (Kwon et al., Proc Natl Acad Sci U S A 104, 10894-9 (2007), Naito et al., Proc Natl Acad Sci U S A 103, 19812-7 (2006)), increased the expression of α-myosin heavy chain (Fig. 2D). In line with these results, cells expressed various muscle transcription factors such as GATA-4, Mef-2C, and Tbx5, while only some donors expressed Nkx2.5 (Fig. 2E). Interestingly, the children-derived cells strongly expressed Islet-1 (Fig. 2E), which was previously shown to define a multipotent primordial cardiovascular progenitor (Laugwitz, supra). Taken together, the blood-derived mesoangioblast-like cells are multipotent and can be directed to differentiate into the 3 distinct cardiovascular cell lineages.

In order to determine the potential functional benefit using these multipotent circulating blood-derived cells for therapeutic applications, human cells were injected in a nude mice model of hind limb ischemia. After 14 days, the recovery of blood flow was significantly greater in mice treated with children-derived cells compared to PBS-treated control mice (Fig. 3A). In addition, cells were injected in mice after induction of myocardial infarction (Table 3).

**Table 3: Characteristics and cardiac functional analysis of nude mice 2 weeks after myocardial infarction (MI) treated with PBS or MAB compared to sham controls**

| | **Sham (n=3)** | **MI+PBS (n=7)** | **MI+MAB (n=6)** |
|---|---|---|---|
| **Age (weeks)** | 12 | 12 | 12 |
| **HR (bpm)** | 684±10 | 525±84 | 520±117 |
| **LVESP (mmHg)** | 99±20 | 93±12 | 86±5 |
| **LVEDP (mmHg)** | 4.8±0.3 | 11.8±2.3 * | 6.4±3.2 ^{##} |
| **Tau (msec)** | 4.5±0.9 | 6.7±1.0 * | 5.5±0.9 ^{#} |

| | | | |
|---|---|---|---|
| HR; heart rate, bpm; beat per minute, LVESP; left ventricle end-systolic pressure, LVEDP; left ventricle end-diastolic pressure * indicates p< 0.01 vs sham. # and ## indicates p< 0.05 and p<0.01 vs MI PBS group, respectively. | | | |

After 2 weeks, cell-treated mice exhibited a smaller infarct size and a significantly improved cardiac function with lower left ventricular end-diastolic pressures and improved diastolic function (Tau) (Fig. 3B/C). The in vivo differentiation of the injected cells was assessed by immunostaining and RT-PCR. Injected cells differentiated to endothelial cells, smooth muscle cells, and cardiomyocytes (data not shown). Moreover, cardiac and endothelial differentiation was confirmed by human specific RT-PCR of troponin T and Tie2, respectively (Fig. 3D). In addition to the capacity of the injected cells to contribute to tissue regeneration in its pure sense by virtue of their differentiation to the cardiovascular cell lineages, cells express and secrete a variety of pro-angiogenic cytokines and cardioprotective factors known to contribute to improved infarct healing.

So far, due to ethical reasons, peripheral blood-derived cells from young donors had only been obtained from children undergoing cardiopulmonary bypass for cardiac surgery. Therefore, we questioned whether the mesoangioblast-like cells had been mobilized upon the stress induced by cardiopulmonary bypass and cardioplegia. Indeed, when we used blood without using cardiopulmonary bypass, the incidence of obtaining mesoangioblast-like cells was reduced (3 out of 10 patients). Moreover, in no incidence could we obtain clonally expandable mesoangioblast-like cells form blood of adults not undergoing cardiopulmonary bypass. However, in adult patients (age 64-83 years) undergoing open heart surgery with cardiopulmonary bypass and cardioplegia, clonally expandable mesoangioblast-like cells were obtained from the blood of 5 out of 5 patients. Of note, the marker profile of all clones resembled mesoangioblasts (CD73⁺ KDR⁺ CD45⁻, Fig. 4A), but was distinct from the previously reported CXCR4⁺ CD34⁺ cell populations shown to be mobilized in this patient cohort (Mieno et al., Circulation 114, 1186-92 (2006)). Thus, these data indicate that cardiopulmonary bypass and cardioplegia during open heart surgery contribute to mobilization of mesoangioblast-like cells into the blood of both children and adults.

In order to identify factors involved in the mobilization of mesoangioblast-like cells, we screened the profile of circulating cytokines before and after cardiopulmonary bypass (Fig. 4B/C). Out of the cytokines tested, hepatocyte growth factor (HGF) was most profoundly up-regulated (2.6±0.2-fold) during cardiopulmonary bypass. Since mesoangioblast-like cells abundantly express the HGF receptor, c-Met (Fig. 4D/E), we tested whether the administration of HGF can mobilize mesoangioblasts-like cells. 24 hours after intravenous injection of 1µg/kg recombinant HGF into 8 weeks old Lewis rats, mesoangioblast-like clones were detected in 4 out of 5 rats, whereas clones were only detected in 4 out of 14 PBS- injected control rats. Moreover, the total number of clones was significantly higher in HGF-treated rats compared to controls (Fig. 4F). The marker profile of the expanded cells was similar to the human mesoangioblast-like cells (Fig. 4G) and CD45⁻CD73⁺KDR⁺ cells were detected by FACS (data not shown). Taken together, HGF appears to be involved in mobilizing mesoangioblast-like cells into the circulating blood.

## Claims

1. A mesoangioblast-like cell, obtained from a subject's blood, as a medicament, wherein the subject has been exposed to hepatocyte growth factor (HGF) or an agent elevating the subject's HGF level.

2. The mesoangioblast-like cell of claim 1, wherein the agent elevating the subject's HGF level is heparin or a functionally active derivate thereof.

3. The mesoangioblast-like cell of claim 1 or 2,
(i) wherein the subject is an adult; and/or
(ii) wherein the subject is a mammal, particularly a human.

4. The mesoangioblast-like cell of any of claims 1 to 3, wherein the mesoangioblast-like cell is **characterized by**
- the presence of CD73 or KDR (VEGF-receptor-2), optionally also the presence of one or more markers selected from the group consisting of Oct3/4, Klf4, c-myc and Sox17; and the absence of CD45, optionally also the absence CD34 or CD133, or
- the presence of KDR, CD73 and CD29 (and optionally also the presence of Oct3/4, Klf4, c-myc and/or Sox 17); and the absence of CD45, optionally also the absence CD34 or CD 133, or
- the presence of CD73, CD13, CD44, CD144, KDR and CD105 and the absence of CD45, CD34 and CD133, or
- the presence of CD73, KDR and telomerase activity and the absence of CD45; or
- especially the presence of CD73 and KDR (VEGF-receptor-2), optionally also the presence CD44, Klf4, Oct3/4 and CD29, and the absence of CD45, optionally also the absence CD34 and CD133, or
- especially the presence of CD105, CD144, KDR, CD13, CD73, CD29 and CD44 and the absence of CD34, CD45, CD 133 and CD 18, or
- most preferably the presence of CD73, KDR (VEGF-receptor-2), Oct3/4, Klf4, c-myc and Sox17, and the absence of CD45, CD34 and CD133.

5. The mesoangioblast-like cell of any of claims 1 to 4,
i) the mesoangioblast-like cell has been isolated at least 1 h to 48 h after the subject's exposure to HGF and/or wherein the subject is exposed to 0.01 to 100 µg/kg bodyweight HGF;
ii) the mesoangioblast-like cell has been isolated at least 1 h to 48 h after the subject's exposure to heparin or a functionally active derivative thereof, e.g. a low-molecular-weight heparin, and/or wherein the subject is exposed to 0.1 to 10 mg/kg bodyweight heparin or a functionally active derivative thereof, e.g. a low-molecular-weight heparin; or
iii) the mesoangioblast-like cell has been isolated at least 1 h to 48 h after the subject's exposure to a combination of (i) HGF and (ii) heparin or a functionally active derivative thereof, e.g. a low-molecular-weight heparin, and/or wherein the subject is exposed to (i) 0.01 to 100 µg/kg bodyweight HGF and (ii) 0.1 to 10 mg/kg bodyweight heparin or a functionally active derivative thereof. e.g. a low-molecular-weight heparin.

6. The mesoangioblast-like cell of any of claims 1 to 5, wherein the mesoangioblast-like cell is capable of differentiating into a mesoderm-derived cell, particularly into one, two or three cardiovascular lineage(s).

7. The mesoangioblast-like cell of any of claims 1 to 6 for the treatment of a cardiovascular disease and/or an ischemic disease, particularly wherein the cardiovascular and/or ischemic disease is selected from the group consisting of myocardial infarction, heart failure and peripheral vascular occlusive disease.

8. A method of isolating a mesoangioblast-like cell from a subject, comprising the steps of:
a) exposing a subject to HGF and/or an agent elevating the subject's HGF level; and
b) isolating a mesoangioblast-like cell mobilized by step a) from the subject.

9. A method of producing a mesoderm-derived cell, comprising the steps of:
a) exposing a subject to HGF and/or an agent elevating the subject's HGF level;
b) isolating a mesoangioblast-like cell mobilized by step a) from the subject; and
c) differentiating the mesoangioblast-like cell into a mesoderm-derived cell.

10. The method of claim 8 or 9, wherein the mesoangioblast-like cell is further defined as in any of claims 2 to 5.

11. The method of any of claims 8 to 10, wherein the mesoderm-derived cell is an endothelial cell, a smooth muscle cell, a cardiomyocyte or an osteoblast.

12. The method of any of claims 8 to 11, wherein the mesoangioblast-like cell isolated in step b) is expanded, particularly clonally expanded.

13. The method of any of claims 9 to 12, wherein the differentiating of step c) is carried out by incubating the mesoangioblast-like cell in the presence of a differentiation factor.

14. Use of a mesoangioblast-like cell for the preparation of a medicament for treating a cardiovascular disease and/or an ischemic disease, wherein the mesoangioblast-like cell has been obtained from a subject exposed to hepatocyte growth factor (HGF) and/or an agent elevating the subject's HGF level, particularly wherein the mesoangioblast is further defined as in any of claims 2 to 7.

15. A method of converting the mesoangioblast-like cell of any of the claims 1 to 6 into an inducible pluripotent stem cell, wherein the level of Sox2 protein in the mesoangioblast-like cell is increased, particularly wherein the increase is mediated by:
a) transfecting the mesoangioblast-like cell of any of claims 1 to 6 with the Sox2 gene and expressing the same;
b) transducing the mesoangioblast-like cell of any of claims 1 to 6 with the Sox2 protein; and/or
c) activating a promoter directing the Sox2 gene expression in the mesoangioblast-like cell of any of claims 1 to 6.
